(19) Europäisches Patentamt
European Patent Office
Office européen des brevets

(11) **EP 3 874 957 A1**

(12) **EUROPEAN PATENT APPLICATION**
published in accordance with Art. 153(4) EPC

(43) Date of publication:
**08.09.2021 Bulletin 2021/36**

(21) Application number: **19877952.2**

(22) Date of filing: **31.10.2019**

(51) Int Cl.:
*A23C 9/13* (2006.01)  *A23L 3/00* (2006.01)
*C08B 30/00* (2006.01)  *A61Q 19/00* (2006.01)
*A23L 5/00* (2016.01)  *A23L 23/00* (2016.01)
*A23L 29/00* (2016.01)  *A23L 29/212* (2016.01)
*A61K 8/73* (2006.01)

(86) International application number:
**PCT/JP2019/042870**

(87) International publication number:
**WO 2020/090994 (07.05.2020 Gazette 2020/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.11.2018 JP 2018206925**

(71) Applicant: **SANWA STARCH CO., LTD.
Kashihara-shi, Nara 634-8585 (JP)**

(72) Inventors:
• **SUNAKO Michihiro
Kashihara-shi, Nara 634-8585 (JP)**
• **UEDA Yasunori
Kashihara-shi, Nara 634-8585 (JP)**
• **MATSUMOTO Noriko
Kashihara-shi, Nara 634-8585 (JP)**

(74) Representative: **Müller-Boré & Partner
Patentanwälte PartG mbB
Friedenheimer Brücke 21
80639 München (DE)**

(54) **RESISTANT STARCH AND PRODUCTION METHOD THEREOF**

(57)    A resistant starch is provided which, when added to a food, does not reduce the flavor of the food. This resistant starch is characterized in that the supernatant, obtained by creating a 33% suspension of the resistant starch in ultrapure water and centrifugally separating for 10 minutes at 2000xg, exhibits electrical conductivity of less than 350 μS/cm. A method for producing a resistant starch is also provided which involves a step for mixing a raw material starch and an acid and subjecting the mixture to conditions in which the resistant starch is generated.

EP 3 874 957 A1

**Description**

[Technical Field]

The present disclosure relates to a resistant starch and a manufacturing method thereof. The present disclosure further relates to food products, cosmetic products, drugs, industrial products, and the like comprising a resistant starch.

[Background Art]

Starches are utilized in a broad range of applications. Chemical modification such as chemical crosslinking using a crosslinker such as epichlorohydrin, glyoxal, or trimetaphosphate is utilized in order to suppress thermal swelling of a starch (Non Patent Literature 1, Non Patent Literature 2, and Non Patent Literature 3). Meanwhile, a method has been proposed, which suppresses thermal swelling of a starch by adding an organic acid salt at 0.1 to 10% to a starch and heating the starch to cause denaturation of the starch (Patent Literature 1).

The Food Sanitation Act considers starches, when processed, as a type of "food additive", i.e., "modified starch", under the "Specifications and Standards for Food, Food Additives, Etc." depending on the processing method. In view of the recent heightened interest in food safety/reliability, consumers who avoid "modified starch" are increasing.

[Citation List]

[Patent Literature]

[PTL 1] Japanese Laid-Open Publication No. 2005-171112

[Non Patent Literature]

[NPL 1] Keiji Kainuma et al., "Studies on Structure and Physico-chemical Properties of Starch", J. Jap. Soc. Starch Sci., Japanese Society of Starch Science, 1975, Vol. 22, No. 3, p.66-71
[NPL 2] Yoshijiro Kihara et al., "Denpun ni Taisuru Arudehido no Sayo (I) [Action of aldehyde on starch (I)]", Denpun Kogyo Gakkaishi, The Japanese Society of Applied Glycoscience, 1962, Vol. 10, No. 1, p.1-6
[NPL 3] Kazuhiro Okuma, "Denpun no Kako to Shokuhin Riyo [Starch modification and use in food products]", Journal of Applied Glycoscinece, The Japanese Society of Applied Glycoscience, 2011, Vol. 1, No. 1, p. 34-38

[Summary of Invention]

[Solution to Problem]

The inventors completed the present invention by discovering that the physical properties/functions of a starch can be controlled by heating the starch in the presence of an acid (e.g., juice). The concept of imparting a swelling suppressing effect by a simple method of contacting a starch with an acid (e.g., juice), which allows the starch to be considered as a food product instead of modified starch, and heating the starch, is a surprising effect that has not been suggested or disclosed.

In one aspect, the present disclosure provides a resistant starch prepared by a manufacturing method comprising the step of mixing a raw material starch with an acid and subjecting the mixture to a condition that generates a resistant starch, and a manufacturing method thereof. Furthermore, the resistant starch of the present disclosure is characterized by an indicator of flavor, i.e., an electrical conductivity (electrical conductivity in the supernatant obtained by suspending the starch in ultrapure water at 33% and centrifuging for 10 minutes at $2000 \times g$), of less than 350 $\mu$S/cm, and enables the manufacture of food and beverage products without deterioration in flavor.

The present disclosure provides, for example, the following items to achieve the objectives described above.

(Item X1)
A swelling suppressed starch, wherein supernatant obtained by suspending the swelling suppressed starch in ultrapure water at 33% and centrifuging for 10 minutes at $2000 \times g$ exhibits an electrical conductivity of less than 350 $\mu$S/cm.
(Item X2)
The swelling suppressed starch of the preceding item, wherein retort resistance is 24% or greater.

(Item X3)

The swelling suppressed starch of any one of the preceding items, wherein thermal resistance is 90% or greater.

(Item X4)

The swelling suppressed starch of any one of the preceding items, wherein acid resistance is 58% or greater.

(Item X5)

A composition comprising the swelling suppressed starch of any one of the preceding items for use as an alternative to a swelling suppressed modified starch.

(Item X6)

A composition comprising the swelling suppressed starch of any one of the preceding items for the manufacture of a retort food product without compromising flavor.

(Item X7)

A composition comprising the swelling suppressed starch of any one of the preceding items for use under a high temperature/high shearing condition.

(Item X8)

A method for manufacturing a swelling suppressed starch, the method comprising the step of mixing a raw material starch with an acid and subjecting the mixture to a condition that generates a swelling suppressed starch.

(Item X9)

The method of any one of the preceding items, wherein the raw material starch is further mixed with an amino acid, a saccharide, or a combination thereof.

(Item X10)

The method of any one of the preceding items, wherein the raw material starch is mixed with an amino acid, a saccharide, and an acid.

(Item X11)

The method of any one of the preceding items, wherein the amino acid, the saccharide, and/or the acid is provided as juice.

(Item X12)

The method of any one of the preceding items, wherein the juice is citrus or drupe juice.

(Item X13)

The method of any one of the preceding items, wherein the juice is from a lemon, a lime, shikuwasa, or an ume.

(Item X14)

The method of any one of the preceding items, wherein the saccharide is a monosaccharide or a disaccharide.

(Item X15)

The method of any one of the preceding items, wherein the saccharide is fructose.

(Item X16)

The method of any one of the preceding items, wherein the amino acid is a neutral amino acid.

(Item X17)

The method of any one of the preceding items, wherein the amino acid is asparagine or cysteine.

(Item X18)

The method of any one of the preceding items, wherein the acid is an organic acid.

(Item X19)

The method of any one of the preceding items, wherein the acid is a citric acid and/or a malic acid.

(Item X20)

The method of any one of the preceding items, wherein a pH of a mixture comprising the raw material starch and the acid is adjusted to a pH of 3 to 7 before subjecting the mixture to the condition that generates the swelling suppressed starch.

(Item X21)

The method of any one of the preceding items, wherein the pH is less than 6.

(Item X22)

The method of any one of the preceding items, further comprising the step of washing the heated product with water after subjecting the mixture to the condition that generates a swelling suppressed starch.

(Item X23)

The method of any one of the preceding items, wherein the condition that generates a swelling suppressed starch comprises a heating step.

(Item X24)

The method of any one of the preceding items, wherein the heating step is performed at 120 to 200°C.

(Item X25)

The method of any one of the preceding items, wherein the heating step is performed at 140 to 180°C.

(Item X26)

The method of any one of the preceding items, wherein the heating step is performed for 40 hours or less.
(Item X27)
A swelling suppressed starch manufactured by the method of any one of the preceding items.
(Item X28)
A composition for manufacturing a swelling suppressed starch, comprising an acid.
(Item X29)
The composition of any one of the preceding items, further comprising an amino acid, a saccharide, or a combination thereof.
(Item X30)
The composition of any one of the preceding items, wherein the amino acid, the saccharide, and/or the acid is provided as juice.
(Item X31)
A food product comprising the swelling suppressed starch of any one of the preceding items.
(Item X32)
The food product of any one of the preceding items, wherein the food product is yogurt, sauce, or a retort product.
(Item X33)
A cosmetic product comprising the swelling suppressed starch of any one of the preceding items.
(Item X34)
An industrial product comprising the swelling suppressed starch of any one of the preceding items.

The present disclosure also provides the following.

(Item 1)
A resistant starch, wherein supernatant obtained by suspending the resistant starch in ultrapure water at 33% and centrifuging for 10 minutes at $2000 \times g$ exhibits an electrical conductivity of less than 350 $\mu$S/cm, and the resistant starch has retort resistance of 20% or greater.
(Item 2)
The resistant starch of the preceding item, wherein the retort resistance is 30% or greater.
(Item 3)
The resistant starch of any one of the preceding items, wherein the retort resistance is 40% or greater.
(Item 4)
The resistant starch of any one of the preceding items, wherein thermal resistance is 90% or greater.
(Item 5)
The resistant starch of any one of the preceding items, wherein acid resistance is 58% or greater.
(Item 6)
A composition comprising the resistant starch of any one of the preceding items for use as an alternative to a swelling suppressed modified starch.
(Item 7)
A composition comprising the resistant starch of any one of the preceding items for the manufacture of a retort food product without compromising flavor.
(Item 8)
A composition comprising the resistant starch of any one of the preceding items for use under a high temperature/high shearing condition.
(Item 9)
A method of manufacturing a resistant starch, the method comprising the steps of:

mixing a raw material starch and an acid with at least one of a saccharide and an amino acid and subjecting the mixture to heating; and
washing a product with water after the step of subjecting the mixture to heating;
wherein the acid is a carboxylic acid.

(Item 10)
The method of any one of the preceding items, wherein the raw material starch is mixed with the acid, the saccharide, and the amino acid.
(Item 11)
The method of any one of the preceding items, wherein the acid, the saccharide, and/or the amino acid is provided as juice.
(Item 12)

The method of any one of the preceding items, wherein a pH of a mixture, when mixing the raw material starch, the acid, and the saccharide, is adjusted to a pH of 3.7 or greater before the step of subjecting the mixture to heating.
(Item 13)
The method of any one of the preceding items, wherein a pH of a mixture, when mixing the raw material starch, the acid, and the amino acid, is adjusted to a pH of 3 or greater before the step of subjecting the mixture to heating.
(Item 14)
The method of any one of the preceding items, wherein a pH of a mixture, when mixing the raw material starch, the acid, the saccharide, and the amino acid, is adjusted to a pH of 3 or greater before the step of subjecting the mixture to heating.
(Item 15)
The method of any one of the preceding items, further comprising the step of selecting a product, wherein supernatant obtained by suspending the product in ultrapure water at 33% and centrifuging the product for 10 minutes at 2000 $\times$ g exhibits an electrical conductivity of less than 350 $\mu$S/cm.
(Item 16)
A resistant starch manufactured by the method of any one of the preceding items.
(Item 17)
A composition for manufacturing a resistant starch, comprising an acid.
(Item 18)
The composition of any one of the preceding items, further comprising an amino acid, a saccharide, or a combination thereof.
(Item 19)
The composition of any one of the preceding items, wherein the amino acid, the saccharide, and/or the acid is provided as juice.
(Item 20)
A food product comprising the resistant starch of any one of the preceding items.
(Item 21)
The food product of any one of the preceding items, wherein the food product is yogurt, sauce, or a retort product.
(Item 22)
A cosmetic product comprising the resistant starch of any one of the preceding items.
(Item 23)
An industrial product comprising the resistant starch of any one of the preceding items.

The present disclosure is intended so that one or more of the features described above can be provided not only as the explicitly disclosed combinations, but also as other combinations thereof. Additional embodiments and advantages of the present disclosure are recognized by those skilled in the art by reading and understanding the following detailed description as needed.

[Advantageous Effects of Invention]

The present disclosure provides a resistant starch with significantly suppressed swelling in comparison to a raw material starch and a manufacturing method thereof. Therefore, the resistant starch of the present disclosure can also be used under a high temperature/high shearing condition. In particular, the present disclosure has improved retort resistance and attains a significant effect in a broad range of food product applications such as retort food products, sauce, and yogurt that require high retort resistance. Furthermore, the resistant starch of the present disclosure can be considered as a "food product" under the Food Sanitation Act of Japan and equivalent legislation in other countries. Since the present disclosure can be used in the manufacture of food products without causing deterioration in flavor that is an issue with swelling suppressed modified starches, the present disclosure can be advantageously used as an alternative to swelling suppressed modified starches. Furthermore, the resistant starch of the present disclosure can be used in the manufacture of various products such as cosmetic products, pharmaceutical products, and industrial products.

[Brief Description of Drawings]

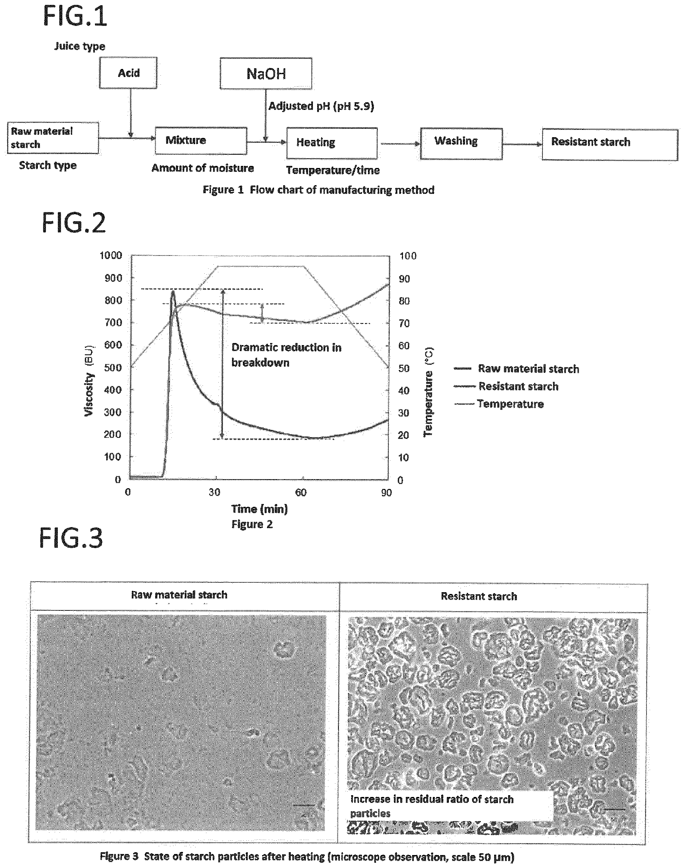

[Figure 1] Figure 1 is a diagram showing an example of a scheme for manufacturing the resistant starch of the present disclosure.
[Figure 2] Figure 2 is a diagram showing viscosity curves of a raw material starch and resistant starch, measured with Viscograph-E in accordance with the method described in Example B. The vertical axis of the graph indicates viscosity or temperature, and the horizontal axis indicates time. The light curve indicates the viscosity curve of the raw material starch, and the dark curve indicates the viscosity curve of the resistant starch. The darkest line segments indicate the temperature changes with the passage of time. The arrows in the graph indicate the range of breakdown. [Figure 3] Figure 3 is a diagram showing microscope pictures of particles of a raw material starch and resistant starch prepared in accordance with the method described in Example B. The left side is a picture of the raw material starch, and the right side is a picture of the resistant starch. The scale bar indicates 50 μm.

[Description of Embodiments]

The present disclosure is described hereinafter while showing the best mode of the invention. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.
The definitions of the terms and/or the detailed basic technology that are particularly used herein are described hereinafter as appropriate.

(Definitions of the terms)

As used herein, the terms "resistant starch", "retort resistant starch", and "swelling suppressed starch" are interchangeably used as terms representing the same meaning and refer to a starch with improved retort resistance relative to a raw material starch. Therefore, "resistance" (level of "swelling suppression" herein) of a starch is typically evaluated herein by measuring retort resistance. The resistant starch herein preferably has retort resistance of 7% or greater, 10% or greater, 16% or greater, 20% or greater, 24% or greater, 30% or greater, 40% or greater, 50% or greater, 60% or greater, 70% or greater, 80% or greater, 90% or greater, or 100% or greater. As used herein, the term "retort resistance", as conventionally used in the art (for instance, the "status of change in viscosity" between "before retort processing" and "after retort processing" is expressed by the term "retort resistance" in "Denpun Seihin no Chishiki [Knowledge on starch products]", author: Reiji Takahashi, Saiwai Shobo, May 1996, p. 187-190), is an indicator indicating the stability of a starch under high temperature and high pressure conditions, and is measured, for example, by the following method. Specifically, 5% by weight of starch on dry basis is suspended in 0.1 M citrate buffer with a pH of 4.5 to prepare a starch slurry. The starch slurry is stirred for 20 minutes in a thermostatic vessel set to 95°C using a disc impeller in a stainless steel beaker. Subsequently, the starch slurry is incubated for 1 hour in a 25°C thermostatic vessel and placed in a container. Retort processing involves processing for 20 minutes at 120°C in an autoclave (Tomy Seiko Co., Ltd.). The resulting starch solution is used to measure the dynamic viscoelasticity with a rheometer (Anton Parr). The viscosity at a shearing rate of 3.16 s$^{-1}$ is extracted, and retort resistance is computed using the following equation.

$$\text{Retort resistance} = (\text{viscosity after retort/viscosity before retort}) \times 100 \ (\%),$$

wherein the viscosity after retort is the viscosity of starch slurry after retort processing for 20 minutes at 120°C, and the viscosity before retort is the viscosity of starch slurry before retort processing.
The resistant starch of the present disclosure typically has a post-retort processing viscosity of 5 to 100,000 mPa·s, preferably 10 to 50,000 mPa·s, preferably 100 to 30,000 mPa·s, preferably 200 to 20,000 mPa·s, preferably 500 to 15,000 mPa·s, and more preferably 1,000 to 10,000 mPa·s. The viscosity cannot be accurately measured for starch slurries resulting in a viscosity of less than 5 mPa·s, preferably less than 10 mPa·s, and more preferably less than 20 mPa·s, or gelatinization (has a viscosity higher than about 200,000 mPa·s, preferably 150,000 mPa·s, and more preferably 100,000 mPa·s) due to the retort processing described above.
Additionally or alternatively, the resistance of a starch (level of swelling suppression herein) can be measured, for example, by the following method (resistance measuring method A):

a) stirring/heating 2 g of starch with 70 g of water in a 85°C thermostatic vessel for 5 minutes; and
b) pouring the product generated in step a into a 100 ml graduated cylinder, filling the cylinder to 100 ml, incubating the solution overnight, and reading the scale.

The volume of precipitated starch layer in step b divided by the weight of the starch (mL/g) is the swelling. A smaller value of swelling indicates greater suppression of swelling of starch particles. Although not wishing to be bound by any theory, the measurement result from the resistance measuring method A is understood to indicate a numerical value that is substantially associated with retort resistance that is typically employed herein. "Resistance" and "swelling suppression" can be evaluated herein by typical retort resistance, and additionally or alternatively by the measurement value from resistance measuring method A, or an indicator for "thermal resistance", "acid resistance", or "shear resistance". Although not wishing to be bound by any theory, it was found in the present disclosure that an indicator indicating swelling is suppressed by subjecting a raw material starch to a condition that generates a resistant starch such as heating or pressurization in the presence of an acid.

As used herein, the term "thermal resistance", as conventionally used in the art (see, for example, "Denpun Seihin no Chishiki [Knowledge on starch products]", author: Reiji Takahashi, Saiwai Shobo, May 1996, p. 122-123), is an indicator indicating the stability of a starch under a high temperature condition and is measured additionally or alternatively as an indicator of "resistance". As used herein, "thermal resistance" is measured by the following method. Specifically, 6% by weight of starch on dry basis is suspended in 0.1 M citrate buffer with a pH of 4.5 to prepare a starch slurry. The temperature of the starch slurry is increased over 13 minutes from 35°C to 95°C, maintained for 10 minutes or 30 minutes, and then cooled to 25°C over 14 minutes with a Rapid Visco Analyzer (Perten Instruments). Immediately thereafter, the dynamic viscoelasticity of the starch slurry is measured with a rheometer (Anton Parr). The viscosity at a shearing rate of 3.16 s$^{-1}$ is extracted, and thermal resistance is computed using the following equation.

```
Thermal resistance (%) = (viscosity upon heating for 30
minutes at 95°C/viscosity upon heating for 10 minutes at
95°C) × 100
```

As used herein, the term "acid resistance", as conventionally used in the art (see, for example, "Denpun Seihin no Chishiki [Knowledge on starch products]", author: Reiji Takahashi, Saiwai Shobo, May 1996, p. 116-118), is an indicator indicating the stability of a starch under acidic conditions and is measured additionally or alternatively as an indicator of "resistance". As used herein, "acid resistance" is measured by the following method. Specifically, 6% by weight of starch on dry basis is suspended in 0.1 M citrate buffer with a pH of 4.5 or a pH of 3.0 to prepare a starch slurry. The temperature of the starch slurry is increased over 13 minutes from 35°C to 95°C, maintained for 10 minutes, and then cooled to 25°C over 14 minutes with a Rapid Visco Analyzer (Perten Instruments). Immediately thereafter, the dynamic viscoelasticity of the starch slurry is measured with a rheometer (Anton Parr). The viscosity at a shearing rate of 3.16 s$^{-1}$ is extracted, and acid resistance is computed using the following equation.

```
Acid resistance = (viscosity at a pH of 3.0/viscosity at a
pH of 4.5) × 100 (%)
```

As used herein, the term "shear resistance" is an indicator indicating the stability of a starch under high shearing conditions and is measured additionally or alternatively as an indicator of "resistance". As used herein, "shear resistance" is measured by the following method. Specifically, 5% by weight of starch on dry basis is suspended in 0.1 M citrate buffer with a pH of 4.5 to prepare a starch slurry. The starch slurry is stirred for 20 minutes in a thermostatic vessel set to 95°C using a disc impeller in a stainless steel beaker. Subsequently, the starch slurry is incubated for 1 hour in a 25°C thermostatic vessel and placed in a container. The shearing processing involves shearing for 2 minutes at 3000 rpm using a homogenizing mixer MARK II (PRIMIX Corporation). The resulting starch solution is used to measure the dynamic viscoelasticity with a rheometer (Anton Parr). The viscosity at a shearing rate of 3.16 s$^{-1}$ is extracted, and shear resistance is computed using the following equation.

```
Shear resistance = (viscosity after shearing/viscosity
before shearing) × 100 (%)
```

As used herein, the terms "electrical conductivity", "conductivity", "electrical conductivity rate", and the like are interchangeably used, and are indicators indicating how readily electricity flows. As used herein, "electrical conductivity" is measured by the following method. Specifically, electrical conductivity can be measured by using a conductivity meter B-173 (Horiba, Ltd.) in supernatant obtained by suspending a target object (e.g., starch) so that it would be 33% by weight ratio in ultrapure water at 25°C and centrifuging for 10 minutes at 2000 × g. A target object is suspended under electrical conductivity measurement conditions so that the object would be 33% (it is understood that denotation of only 33% also refers to 33% in dry basis herein, as is well known in the art) in dry basis by weight ratio in ultrapure water at 25°C. Since physical property values of starch are also widely evaluated by measuring parameters while being dissolved in water (see, for example, "Standard Tables of Food Composition in Japan -2015- (Seventh Revised Edition) (Potatoes and starches/(starches)/Potato starch)" (https://fooddb.mext.go.jp/details/details.pl?ITEM_NO=2_020 34_7), Standard Tables of Food Composition in Japan -2015-(Seventh Revised Edition) analysis manual (http://www.mext.go.jp/component/a_menu/science/detail/_ic sFiles/afieldfile/2016/03/25/1368932_01_1.pdf), "Denpun Kagaku Jikkenho [Starch Science Experimental Method]", authors: Shigeo Suzuki and Michitoku Nakamura, Asakura Publishing, October 1979, p. 279-283, and the like), the "electrical conductivity" measured by the measurement method described above is a physical property value that is unique to the starch itself. Although not wishing to be bound by any theory, electrical conductivity is considered to be related to flavor (e.g., Japanese Patent No. 3513047 and Japanese Laid-Open Publication No. 2016-178892), and the resistant starch of the present disclosure has a significantly improved electrical conductivity relative to a raw material starch and modified starch, so that the flavor of the resistant starch of the present disclosure is understood to be improved. In fact, as far as the inventors are aware, it is known that a starch with a high electrical conductivity has poor flavor, and a starch with a low electrical conductivity has good flavor. This can also be found from sensory evaluation or the like. The electrical conductivity is preferably less than 350 µS/cm in the present disclosure.

As used herein, the term "viscosity behavior" has the same meaning as "amylograph" or the like, referring to the viscosity over time (and data thereof) measured using a viscometer such as Viscograph®-E available from Brabender. The viscosity behavior herein is measured by suspending 6% by weight of starch on dry basis into distilled water to prepare a starch slurry and recording the changes in viscosity over time when the temperature of the starch slurry is increased over 30 minutes from 50°C to 95°C, maintained for 30 minutes, and then cooled to 50°C over 30 minutes with a viscometer such as Viscograph-E (Brabender GmbH & Co KG). The maximum viscosity in the present disclosure refers to the maximum value on a viscosity curve upon heating or heating, maintaining, and gelatinizing the starch slurry at the concentration described above with the thermal history described above. However, when the degree of swelling suppression is high, the peak viscosity may be lost and the viscosity history continuously increases in some cases (specific cases where the heating temperature/time is 180°C/4 hours or 200°C/1 hour, or the pH of a mixture of a raw material starch and acid is 4.51). Further, the minimum viscosity refers to the lowest viscosity after the manifestation of the peak viscosity. Breakdown refers to the difference between the maximum viscosity and minimum viscosity.

As used herein, the terms "retort food product" or "retort product" generally refer to prepared food product that is packaged in a container (limited to those with air-tight and light proof properties) molded from a plastic film, metal foil, or combination of multiple layers thereof into a shape of a pouch or other shapes, and sealed by thermal fusion, pressurized, heated, and sterilized. "Retort food product" in the present disclosure includes those described above, as well as food products that are placed in an air-tight container, sealed, then pressurized, heated, and sterilized. Examples thereof include any food products packaged in a container such as a can or bottle.

As used herein, the term "high temperature/high shearing condition" refers to a high temperature and/or high shearing condition such as a condition where a temperature is 95°C or higher and/or stirring is performed at 3000 rpm or greater. Usability under a high temperature/high shearing condition can be specifically tested by a method such as measuring thermal resistance by measuring the viscosity after heating and maintaining a starch for 30 minutes at 95°C, measuring shear resistance by measuring the viscosity after stirring a starch for 2 minutes at 3000 rpm using a homogenizing mixer, or measuring retort resistance by measuring the viscosity after subjecting a starch to retort processing for 20 minutes at 120°C.

As used herein, the term "raw material starch" refers to any starch that can be used as a raw material for manufacturing a resistant starch. "Raw material starch" is not particularly limited herein, as long as it is used in applications such as food products, cosmetic products, pharmaceutical products, or industrial products. Examples thereof include corn starch, waxy corn starch, high-amylose corn starch, potato starch, tapioca starch, wheat starch, rice starch, sago starch, sweet potato starch, green pea starch, mung bean starch, and such starches subjected to some type of processing (e.g., heat-moisture treatment). These starches can be used independently or as a combination of a plurality of starches. The "raw material starch" herein is not a modified starch.

As used herein, the term "swelling suppressed modified starch" refers to modified starch with suppressed swelling of the starch. "Modified starch" herein refers to starch acetate, monostarch phosphate, hydroxypropyl starch, acetylated distarch adipate, starch sodium octenylsuccinate, oxidized starch, acetylated oxidized starch, distarch phosphate, acetylated distarch phosphate, phosphated distarch phosphate, hydroxypropylated distarch phosphate, sodium starch glycolate starch, sodium starch phosphate starch, other starches that are designated as, and required by law to be displayed as, "modified starch" under the Food Sanitation Act of Japan, or starches equivalent to "modified starch" under similar legislation in other countries.

As used herein, the term "acid" is used in the meaning that is generally used in the art, referring to a chemical species that gives proton ($H^+$) or receives an electron pair. "Acid" encompasses inorganic acids and organic acids. "Inorganic acid" is a collective term for acids of an inorganic compound, which is a compound consisting of hydrogen and one nonmetal element or a group thereof. Examples of inorganic acids include hydrochloric acid, nitric acid, boric acid, sulfuric acid, carbonic acid, phosphoric acid, and the like. "Organic acid" is a collective term for acids of an organic compound. "Organic acid" includes carboxylic acids having a carboxy group and sulfonic acids having a sulfo group. Carboxylic acid is further classified by the number of carboxy groups within a molecule. Examples thereof include monocarboxylic acids (e.g., formic acid, acetic acid, propionic acid, quinic acid, and the like), dicarboxylic acids (e.g., oxalic acid, malonic acid, succinic acid, malic acid, tartaric acid, and the like), tricarboxylic acids (e.g., citric acid, aconitic acid, and the like), and the like. Carboxylic acid further includes sugar acids (e.g., ascorbic acid, gluconic acid, glucuronic acid, tartaric acid, citric acid, and the like), which are compounds prepared from substituting an oxygen group of a monosaccharide with a carboxylic acid. In the present disclosure, organic acid, more preferably carboxylic acid is used, and organic acid contained in juice (e.g., citric acid, malic acid, or the like) can be particularly preferably used, but the acid is not limited thereto.

As used herein, the term "amino acid" refers to an organic compound having an amino group ($NH_2$) or imino group ($>C=NH$) and a carboxyl group (COOH). As used herein, "amino acid" includes organic compounds having an amino group ($NH_2$) or imino group and a carboxyl group (COOH) in a salt form, the compounds in which an amino group, imino group, and/or carboxyl group is utilized for a bond (e.g., amide bond or ester bond) (those in a protein), and the compounds comprising an imino group in place of an amino group (e.g., proline). In some embodiments, "amino acid" is an $\alpha$-amino acid with an amino group and a carboxyl group binding to the same carbon atom. As used herein, "amino acid" encompasses amino acids mainly constituting a protein found within an organism (alanine, cysteine, aspartic acid, glutamic acid, phenylalanine, glycine, histidine, isoleucine, lysine, leucine, methionine, asparagine, proline, glutamine, arginine, serine, threonine, valine, tryptophan, and tyrosine). Amino acids can be classified based on the type of side chain, including basic amino acids (lysine, arginine, histidine, and tryptophan), acidic amino acids (aspartic acid and glutamic acid), neutral amino acids (asparagine, alanine, isoleucine, glycine, glutamine, cysteine, threonine, serine, tyrosine, phenylalanine, proline, valine, methionine, and leucine), aromatic amino acids (tyrosine, phenylalanine, tryptophan, and histidine), hydroxyl amino acids (serine, threonine, and tyrosine), sulfur containing amino acids (cysteine and methionine), and the like. Although not wishing to be bound by any theory, asparagine, aspartic acid, cysteine, or the like can be advantageously used.

As used herein, the term "saccharide" is used in the meaning that is generally used in the art, referring to a compound having a ketone group ($>C=O$) or aldehyde group (-CHO) and having a plurality of hydroxyl groups (-OH). "Saccharide" can be classified into monosaccharide, disaccharide, oligosaccharide, polysaccharide, or the like in accordance with the number of monosaccharide molecules that are contained. Unless specifically noted otherwise, "saccharide" is generally interpreted herein to exclude "starch". Examples of monosaccharides include glucose, galactose, mannose, fructose, and the like. Examples of disaccharides include lactose, sucrose, maltose, and the like. Examples of polysaccharides include glycogen and the like, which can be used in the present disclosure. Preferably, a monosaccharide or disaccharide is used.

As used herein, the term "juice" refers to the sap from a fruit or vegetable. As used herein, "juice" can be used for straight juice and juice that is produced by concentration. An ingredient other than the juice (e.g., preservative, additive, saccharide, honey, salt, or the like) can be added. The juice used in the present disclosure is not particularly limited. For example, ume juice, lime juice, shikuwasa juice, grapefruit juice, orange juice, apple juice, lemon juice, melon juice, grape juice, kiwi juice, mango juice, pineapple juice, lychee juice, pear juice, peach juice, cherry juice, watermelon juice, acerola cherry juice, yuzu juice, sudachi juice, kabosu juice, blackcurrant juice, red raspberry juice, or the like can be used. The juice used in the present disclosure can be used independently or as a mixture of two or more types of juice. The juice used in the present disclosure is preferably, but is not limited thereto, juice with a total citric acid, malic acid, and tartaric acid content in the juice of 0.30% or greater. It is understood that mixtures of juice that can impart retort resistant to the same degree as such juice, mixtures of juice and other ingredients, and artificially constituted juice (e.g., including concentrate of juice and the like) are encompassed within the scope of the present disclosure, as long as they have the same function. More specifically, juice of citrus (e.g., lime, shikuwasa, grapefruit, orange, lemon, yuzu, sudachi, kabosu, or the like), fruit of the genus Prunus in the family Rosaceae (e.g., apricot, ume, cherry, plum, peach, prune, nectarine, or the like), or drupe (e.g., ume, mango, peach, cherry, or the like) is preferable. Representative examples thereof include juice of lemon, lime, shikuwasa, or ume.

(Preferred embodiments)

Preferred embodiments of the present disclosure are described below. Embodiments described below are provided to facilitate the understanding of the present disclosure. It is understood that the scope of the present invention should not be limited to the following descriptions. Thus, it is apparent that those skilled in the art can make appropriate modifications within the scope of the present invention by referring to the descriptions herein. It is also understood that the following embodiments of the present disclosure can be used independently or as a combination thereof.

Each of the embodiments described below provides a comprehensive or specific example. The numerical values, shapes, materials, constituent elements, steps, order of steps, and the like in the following embodiments are one example, which are not intended to limit the Claims. Further, the constituent elements in the following embodiments that are not recited in the independent claims showing the most superordinate concept are described as an optional constituent element.

(Resistant starch)

In one aspect, the present disclosure provides a resistant starch, wherein supernatant obtained by suspending the resistant starch in ultrapure water at 33% and centrifuging for 10 minutes at 2000 × g exhibits an electrical conductivity (also referred to as "electrical conductivity for flavor" herein) of less than 350 μS/cm. Electrical conductivity for flavor is widely used as an indicator indicating the flavor of a starch and is also used in the present disclosure as an indicator of flavor. Although not wishing to be bound by any theory, it has been found that the body of flavor is felt when the electrical conductivity for flavor is less than 350 μS/cm, but this numerical value can vary. Therefore, representative examples of the electrical conductivity for flavor of the resistant starch of the present disclosure include less than 350 μS/cm, less than 300 μS/cm, and less than 250 μS/cm. The resistant starch of the present disclosure exhibits an excellent property of suppressed swelling and can be used without compromising flavor when used in food products.

The resistant starch of the present disclosure is also advantageous in that the starch can be considered as a "food product" under the Food Sanitation Law of Japan and equivalent legislation in other countries. The resistant starch of the present disclosure does not fall under chemically processed starches (i.e., acetylated distarch adipate, acetylated oxidized starch, acetylated distarch phosphate, starch sodium octenylsuccinate, starch acetate, oxidized starch, hydroxypropylated distarch phosphate, hydroxypropyl starch, distarch phosphate, monostarch phosphate, and phosphated distarch phosphate) that are required by law to be displayed as "food additive" as "modified starch" under the Food Sanitation Act of Japan as of the filing of the present application. Therefore, the resistant starch of the present disclosure attains an advantageous effect in terms of achieving an effect of suppressed swelling without the chemical processing described above.

(Method of manufacturing resistant starch)

The resistant starch of the present disclosure is manufactured by mixing a raw material starch with an acid and subjecting the mixture to a condition that generates a resistant starch. The raw material starch described above is not particularly limited, as long as the starch is used in applications for food products, cosmetic products, pharmaceutical products, or industrial products. The raw material starch can be manufactured from a plant, or a commercially available starch can be used. Unprocessed starches can be typically used as the raw material starch, but processed starch such as heat-moisture treated starch can also be used. When using a processed starch, such a starch that is not classified as a modified starch is utilized. Preferred examples of raw material starches include waxy corn starch, tapioca starch, rice starch, and glutinous rice starch.

A raw material starch and an acid can be mixed in the manufacturing method described above by a commonly used method. Examples of the mixing method include, but are not limited to, a method of stirring and mixing with an apparatus such as a ribbon mixer or Nauta mixer.

In some embodiments, the acid in the manufacturing method described above is an organic acid. In a preferred embodiment, the acid is a citric acid and/or a malic acid. The amount of acid added to a starch is preferably 0.022 to 2.2% by weight, more preferably 0.088 to 0.88% by weight, and most preferably 0.22 to 0.44% by weight, in terms of percent solids by weight. These acids can also be provided as juice or as an acid extracted from juice or acid containing preparation. Therefore, in one embodiment, an acid utilized in the present disclosure can be preferably any acid contained in juice.

In some embodiments, the acid, amino acid, and saccharide in the manufacturing method described above are provided as juice. The amount of juice added to a starch, in equal units, is preferably 0.5 to 50% by weight, more preferably 2.0 to 20% by weight, and most preferably 5.0 to 10% in terms of percentage solids by weight. These acids can also be provided as juice or as an acid extracted from juice or acid containing preparation. Therefore, in one embodiment, an acid utilized in the present disclosure can be preferably any acid contained in juice.

In some embodiments, a raw material starch in the manufacturing method described above is also mixed with an amino acid, a saccharide, or a combination thereof in addition to an acid. In one embodiment, the type of amino acid is preferably a neutral amino acid and an acidic amino acid, and particularly preferably asparagine, aspartic acid, glutamine, or cysteine. The type of saccharide is preferably a monosaccharide or disaccharide, and particularly preferably fructose. The amount of amino acid added is preferably 0.002 to 0.2% by weight, more preferably 0.008 to 0.08% by weight, and most preferably 0.02 to 0.04% by weight with respect to starch, in terms of percent solids by weight. The amount of saccharide is preferably 0.0025 to 0.25% by weight, more preferably 0.01 to 0.1% by weight, and most preferably 0.025 to 0.05% by weight with respect to starch, in terms of percent solids by weight. When comprising an amino acid, a saccharide, or a combination thereof, this can be provided as juice, or provided as a mixture of an acid and an amino acid, a saccharide, or a combination thereof extracted from juice, or a preparation containing an acid, amino acid, a saccharide, or a combination thereof.

In some embodiments where a raw material starch is mixed with an acid, amino acid, and saccharide, the acid, amino acid, and saccharide are provided as juice. The type of juice is not particularly limited, but juice of citrus or drupe is preferable. In one preferred embodiment, the juice is juice of a lemon, lime, shikuwasa, or ume, or a fruit equivalent thereto.

Examples of conditions that generate a resistant starch in the manufacturing method used herein include, but are not limited to, heating, pressurizing, and a combination thereof. In some embodiments, a condition that generates a resistant starch in the manufacturing method described above is subjecting to heating. Heating can be performed by a commonly used method. Specifically, a shelf dryer, a fluidized-bed roaster, or the like can be used. Heating is particularly preferably heating using a heater using indirect heating such as a paddle dryer. Such a method is capable of uniform and efficient heating.

In some embodiments, the heating that can be utilized in the present disclosure is performed at a temperature of 120 to 200°C, preferably 140°C to 200°C, and more preferably 140°C to 180°C. Heating at a temperature lower than 120°C results in a low reaction rate with poor manufacturing efficiency, while a temperature of 200°C or higher results in degradation of starch molecules or coloration of starch, so that such temperatures are not preferred because a desired resistant starch cannot be obtained. The time of heating is dependent on the temperature of heating, but is 1 to 40 hours, particularly preferably 1 to 4 hours. A short time of heating results in low extent of reaction, while a long time of heating results in degradation of starch molecules or coloration of starch, so that such times are not preferred because a desired resistant starch cannot be obtained.

In a certain embodiment, a mixture of a starch and an acid does not need to be processed to be anhydrous by preliminary drying prior to a condition that generates a resistant starch, and can be subjected directly to a condition that generates a resistant starch. The amount of moisture in a mixture of a starch and an acid is preferably 15 to 35%, more preferably 20 to 30%, and particularly preferably 25 to 30%. If the amount of moisture is lower than 15%, the efficiency of generating a resistant starch would decrease, while at an amount greater than 35%, the line compatibility would deteriorate significantly. However, the present disclosure is not limited to such specific numerical values.

In some embodiments, the manufacturing method described above further comprises the step of adjusting a pH of a mixture comprising a raw material starch and an acid before subjecting the mixture to a condition that generates a resistant starch. The pH of a mixture of a raw material starch and an acid is generally a pH of 3 to 8. The upper limit can be a pH of 8 or less, pH of less than 8, pH of 7 or less, pH of less than 7, pH of 6 or less, pH of less than 6, or the like. The lower limit can be a pH of 3 or greater, pH of 3.7 or greater, pH of 4 or greater, pH of 5 of greater, pH of 6 or greater, or the like. In a certain embodiment, the pH is 4 to 7. In another embodiment, the pH is 6 to 7. In one aspect, pH of a mixture, when mixing the raw material starch, the acid, and the saccharide, is adjusted to a pH of 3.7 or greater before the step of subjecting the mixture to heating. In another aspect, pH of a mixture, when mixing the raw material starch, the acid, and the amino acid, is adjusted to a pH of 3 or greater before the step of subjecting the mixture to heating. In still another aspect, pH of a mixture, when mixing the raw material starch, the acid, the saccharide, and the amino acid, is adjusted to a pH of 3 or greater before the step of subjecting the mixture to heating. If the pH of a mixture of a raw material starch and an acid is lower than 4, a starch would be degraded under a condition that generates a resistant starch. If the pH is 8 or greater, a significant difference in the extent of suppression of swelling would not be observed.

In some embodiments, the manufacturing method described above further comprises the step of washing the product (i.e., resistant starch) resulting from a condition that generates a resistant starch with water, after subjecting the resistant starch to the condition. Although not wishing to be bound by any theory, the washing step attains an advantageous effect because the washing step removes juice components and alkaline impurities in the product and bleaches the product to improve the electrical conductivity of the product and the flavor. Although not wishing to be bound by any theory, the effect of improving flavor by washing with water is an unexpected effect found in the present disclosure.

In some embodiments, the present disclosure provides a resistant starch manufactured by the manufacturing method described above.

(Application of resistant starch)

In one aspect, the resistant starch of the present disclosure can be used as an alternative to a swelling suppressed modified starch. Thus, the present disclosure provides a composition for use as an alternative to a swelling suppressed modified starch. In one embodiment, examples of swelling suppressed modified starches that can be substituted with the resistant starch of the present disclosure include, but are not limited to, esterified, etherified, or oxidized starches and starches obtained from combining these reactions (e.g., starch acetate, monostarch phosphate, hydroxypropyl starch, acetylated distarch adipate, starch sodium octenylsuccinate, oxidized starch, acetylated oxidized starch, distarch phosphate, acetylated distarch phosphate, phosphated distarch phosphate, hydroxypropylated distarch phosphate, sodium starch glycolate starch, sodium starch phosphate starch, and the like) .

In another aspect, the resistant starch of the present disclosure can be used for the manufacture of a retort food product without compromising flavor. Thus, the present disclosure provides a composition for the manufacture of a retort food product without compromising flavor.

In another aspect, the resistant starch of the present disclosure can be used under a high temperature/high shearing condition. Thus, the present disclosure provides a composition for use under a high temperature/high shearing condition. Therefore, the resistant starch of the present disclosure can be used even under harsh conditions (e.g., use in some retort food products or the like) where a raw material starch could not be used.

In another aspect, the resistant starch of the present disclosure can be used in the manufacture of a food product. Thus, the present disclosure provides a food product comprising the resistant starch of the present disclosure. The "food product" herein has the meaning that is routinely used in the art, referring to any food (including beverages) that can be consumed by humans. One embodiment thereof includes a processed product. The resistant starch of the present disclosure is particularly useful in a food product for which flavor is of importance. A food product manufactured by using the resistant starch of the present disclosure is preferably, but is not limited to, yogurt, sauce, or retort product. Examples of food products of the present disclosure include normal food products as well as functionally enhanced food products such as food for specified health uses, food with function claims, food with nutrient function claims, and food claiming equivalent or similar function in other countries.

In another aspect, the resistant starch of the present disclosure can also be used in the manufacture of a cosmetic product. Thus, the present disclosure provides a cosmetic product comprising the resistant starch of the present disclosure. "Cosmetic product" herein has the meaning that is routinely used in the art, referring to a substance intended to be applied, sprayed, or used in other similar methods on the body in order to clean, beautify, increase the attractiveness, or change the appearance of the human body or maintain the health of skin or hair, with mitigated effect on the human body. The resistant starch of the present disclosure has swelling suppressed, so that the resistant starch is particularly useful as a thickener and suspension that can be used in foams, creams, and lotions.

In another aspect, the resistant starch of the present disclosure can also be used in the manufacture of an industrial product. Thus, the present disclosure provides an industrial product comprising the resistant starch of the present disclosure. "Industrial product" herein has the meaning that is routinely used in the art, referring to any article of manufacture associated with the step of consuming a raw material to produce a product. The resistant starch of the present disclosure can be utilized in various industries such as papermaking, adhesives, fibers, construction materials, fertilizers, drugs, casting, rubber, and leather.

In one embodiment, the resistant starch of the present disclosure can also be used in the manufacture of a drug. Thus, the present disclosure provides a drug comprising the resistant starch of the present disclosure. The starch can be used in anything that is subjected to a regulation under the legislation of each country as a drug (e.g., pharmaceutical products, quasi-drugs, regenerative medicine products, supplements, and the like).

(Note)

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the each document is specifically described in its entirety.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present invention is not limited to the embodiments and Examples specifically described herein and is limited only by the scope of claims.

[Examples]

The Examples of the present disclosure are described hereinafter. It is understood that the denotation of % in the Examples indicate % by weight (w/w%), unless specifically noted otherwise.

(Example A: Preparation of resistant starch)

The raw material starches used in the Examples were obtained from the following suppliers: Sanwa Starch Co., Ltd., Nagata Group Holdings, Ltd., THAI WAH (Thailand), JA Kiyosatocho, Joetsu Starch Co., Ltd., and J-Oil Mills, Inc. The juice used in the Examples was obtained from the following suppliers: Nakano BC Co., Ltd., Megmilk Snow Brand Co., Ltd., Ogawa & Co., Ltd., Pokka Sapporo Food & Beverage Ltd., and JA Okinawa. An acid containing preparation was obtained by mixing 5 parts of juice and 10 parts of water, in equal units. 100 parts of raw material starch was mixed with 15 parts of acid containing preparation in terms of mass. The pH was adjusted using sodium hydroxide. The mixture was heated for 4 hours at 150°C using a shelf dryer (ESPEC Corp.) Subsequently, the mixture was washed twice with 200 parts of water, dehydrated, and dried to obtain a resistant starch (Figure 1) .

(Example B: Viscosity behavior of resistant starch and state of starch particles)

(Experimental approach)

A resistant starch prepared in accordance with Example A using a waxy corn starch as the raw material starch, and a raw material starch were suspended in distilled water so that each would be 6% by weight on dry bases to obtain a starch slurry. The temperature of the starch slurry was increased over 30 minutes from 50°C to 95°C, maintained for 30 minutes, and then cooled to 50°C over 30 minutes while recording the viscosity over time with Viscograph-E (Brabender GmbH & Co KG). Furthermore, the state of the starch after heating was observed with a microscope.

(Results)

Figure 2 shows the results of the viscosity behavior of Example B. The results of Figure 2 demonstrated that the resistant starch of the present disclosure had a significantly reduced breakdown value relative to the raw material starch, and swelling of the starch was suppressed significantly. Furthermore, the raw material starch swelled after heating such that the morphology of the starch had changed, whereas the morphology of the resistant starch was maintained in the results of the microscope observation of starches shown in Figure 3, thus demonstrating that swelling of the resistant starch was suppressed significantly.

(Example C: Relationship between type of raw material starch and property of suppressed swelling)

(Experimental approach)

Resistant starches were prepared by the same method as the method described in Example A, except for using corn starch, tapioca starch, rice starch, glutinous rice starch, potato starch, and waxy corn starch as the raw material starches, using ume juice as the acid containing preparation, and mixing 100 parts of the raw material starch with 5 parts or 15 parts of the acid containing preparation in terms of mass. Subsequently, thermal resistance, acid resistance, shear resistance, retort resistance, and electrical conductivity of the raw material starches and resistant starch were measured.

(Results)

Table C shows results of Example C. The resistant starch prepared using corn starch as the raw material starch was demonstrated to exhibit excellent thermal resistance, acid resistance, and retort resistance, and exhibit an excellent property of suppressed swelling. The resistant starch also had low electrical conductivity and excellent flavor. Meanwhile, thermal resistance and retort resistance of the raw material corn starch could not be measured due to gelatinization, and the starch had a poor property of suppressed swelling. Resistant starches prepared by using other raw material starches also yielded the same result. Potato starch had retort resistance of 24% when mixed with 5 parts of acid containing preparation, while retort resistance increased to 70% when mixed with 15 parts of acid containing preparation. Therefore, the manufacturing method of the present disclosure was demonstrated to be applicable to any type of raw material starch.

[Table C]

Table C. Difference in starch types

| Example | Starch type | Amount of juice added (%) | Thermal resistance (%) | Acid resistance (%) | Shearresistance (%) | Retort resistance (%) | Electrical conductivity (μS/cm) |
|---|---|---|---|---|---|---|---|
| 1 | Corn starch | 5 | 163 | 161 | 77 | 289 | 187 |
| 2 | Tapioca starch | 5 | 99 | 67 | 82 | 71 | 250 |
| 3 | Rice starch | 5 | 158 | 118 | 75 | 66 | 260 |
| 4 | Glutinous rice starch | 5 | 93 | 62 | 93 | 48 | 260 |
| 5 | Potato starch | 5 | 90 | 61 | 38 | 24 | 187 |
| 6 | Potato starch | 15 | 105 | 89 | 79 | 70 | 240 |
| 7 | Waxy corn starch | 5 | 121 | 85 | 84 | 73 | 220 |

| Comparative Example | Starch type | Amount of juice added (%) | Thermal resistance (%) | Acid resistance (%) | Shearresistance (%) | Retort resistance (%) | Electrical conductivity (μS/cm) |
|---|---|---|---|---|---|---|---|
| 1 | Raw material corn starch | - | ND | 65 | ND | ND | 161 |
| 2 | Raw material tapioca starch | - | 82 | 15 | 8 | 4 | 430 |
| 3 | Raw material rice starch | - | 87 | 26 | 72 | 6 | 520 |
| 4 | Raw material glutinous rice starch | - | 75 | 17 | 13 | 5 | 530 |
| 5 | Raw material potato starch | - | 66 | 27 | 5 | 1 | 151 |
| 6 | Raw material waxy corn starch | - | 73 | 14 | 15 | 7 | 147 |

(Example D: Relationship between juice type and property of suppressed swelling)

(Experimental approach)

Resistant starches were prepared using a waxy corn starch as a raw material starch by the same method as the method described in Example A, except for the type of juice, using lime, shikuwasa, grapefruit, orange, apple, lemon, and ume juice as the acid containing preparation, and mixing 100 parts of the raw material starch with 5 parts or 15 parts of the acid containing preparation in terms of mass. Subsequently, thermal resistance, acid resistance, shear resistance, retort resistance, and electrical conductivity of the raw material waxy corn starch and resistant starches prepared using each acid containing preparation were measured.

(Results)

Table D shows results of Example D. Resistant starches prepared using lime, shikuwasa, lemon, and ume juice as the acid containing preparation were demonstrated to exhibit excellent thermal resistance, acid resistance, shear resistance, and retort resistance, and exhibit an excellent property of suppressed swelling. The resistant starch prepared using apple juice as the acid containing preparation had retort resistance of 16% when mixed with 5 parts of acid containing preparation, but retort resistance increased to 34% when mixed with 15 parts of acid containing preparation. The resistant starch also had low electrical conductivity and excellent flavor. Meanwhile, thermal resistance, acid resistance, shear resistance, and retort resistance of the raw material waxy corn starch were lower than those of resistant starches prepared using any of the juice.

[Table D]

Table D. Difference in juice type

| Example | Juice type | Amount of juice added (%) | Thermal resistance (%) | Acid resistance (%) | Shear resistance (%) | Retort resistance (%) | Electrical conductivity (μS/cm) |
|---|---|---|---|---|---|---|---|
| 1 | Lime | 5 | 109 | 79 | 105 | 113 | 320 |
| 2 | Shikuwasa | 5 | 108 | 91 | 94 | 82 | 230 |
| 3 | Grapefruit | 5 | 89 | 73 | 66 | 44 | 112 |
| 4 | Orange | 5 | 89 | 74 | 72 | 45 | 109 |
| 5 | Apple | 5 | 86 | 64 | 39 | 16 | 101 |
| 6 | Apple | 15 | 97 | 81 | 65 | 34 | 131 |
| 7 | Lemon | 5 | 100 | 84 | 109 | 91 | 260 |
| 8 | Ume | 5 | 121 | 85 | 84 | 73 | 220 |
| Comparative Example | | Amount of juice added (%) | Thermal resistance (%) | Acid resistance (%) | Shear resistance (%) | Retort resistance (%) | Electrical conductivity (μS/cm) |
| 1 | Raw material corn starch | - | 73 | 14 | 15 | 7 | 147 |

(Example E: Relationship between pH of juice and property of suppressed swelling

(Experimental approach)

Resistant starches were prepared using ume juice as the acid containing preparation and using a waxy corn starch as the raw material starch in accordance with Example A. In this Example, a step of adjusting the pH to 3.42 to 7.20 (Examples 1 to 8) or 2.48 and 8.22 (Comparative Examples 1 and 2) using sodium hydroxide, before heating, on a mixture of the raw material starch and acid containing preparation was added. Subsequently, thermal resistance, acid resistance, shear resistance, retort resistance, and electrical conductivity of the resistant starches prepared by adjusting to each pH were measured. The baseline viscosity (mPa·s) indicates the viscosity before retort processing.

(Results)

Table E shows the results of Example E. Resistant starches prepared by adjusting a mixture of a raw material starch and acid containing preparation to the pH of 3.42 to 7.20 were demonstrated to exhibit excellent thermal resistance, acid resistance, shear resistance, retort resistance, and baseline viscosity and exhibit an excellent property of suppressed swelling. The resistant starches also had a low electrical conductivity and excellent flavor.

[Table E]

Table E. Difference in pH before heating

| Example | pH before heating | Thermal resistance (%) | Acid resistance (%) | Shear resistance (%) | Retort resistance (%) | Baseline viscosity (mPa·s) | Electrical conductivity (μS/cm) |
|---|---|---|---|---|---|---|---|
| 1 | 3.42 | 112 | 100 | 116 | 64 | 1162 | 135 |
| 2 | 4.51 | 135 | 133 | 88 | 180 | 2147 | 150 |
| 3 | 5.02 | 129 | 114 | 101 | 132 | 3444 | 200 |
| 4 | 5.94 | 126 | 97 | 103 | 82 | 5851 | 210 |
| 5 | 6.19 | 121 | 85 | 84 | 73 | 5689 | 220 |
| 6 | 6.32 | 111 | 81 | 51 | 68 | 6000 | 210 |
| 7 | 6.75 | 91 | 72 | 45 | 62 | 6585 | 200 |
| 8 | 7.20 | - | - | - | 50 | 7000 | 200 |
| Comparative Example | pH before heating | Thermal resistance (%) | Acid resistance (%) | Shear resistance (%) | Retort resistance (%) | Baseline viscosity (mPa·s) | Electrical conductivity (μS/cm) |
| 1 | 2.48 | - | - | - | 3 | 312 | 126 |
| 2 | 8.22 | - | - | - | 62 | 5965 | 220 |

EP 3 874 957 A1

(Example F: Relationship between heating condition and property of suppressed swelling)

(Experimental approach)

Resistant starches were prepared using ume juice as the acid containing preparation and using waxy corn starch as the raw material starch. The method of preparing a resistant starch is the same as the method described in Example A, except for changing the heating condition to 100°C, 120°C, 150°C, 180°C, or 200°C and 1 hour, 4 hours, 20 hours, or 40 hours. Subsequently, thermal resistance, acid resistance, shear resistance, retort resistance, and electrical conductivity of resistant starches and starches prepared by heating for 4 hours, 20 hours, or 40 hours at 100°C were measured.

(Results)

Table F shows the results of Example F. The resistant starches prepared by heating for 4 hours at 150°C and 180°C exhibited an excellent effect of suppressed swelling, thus demonstrating that heating for 4 hours at 150°C attains a practical and excellent effect from the viewpoint of especially shear resistance and electrical conductivity. The starches also had a low electrical conductivity and excellent flavor. While data is not shown, resistant starches prepared by heating for 20 to 40 hours at 100°C also had improved acid resistance and retort resistance and a property of suppressed swelling.

[Table F]

Table F. Difference in heating temperature/time

| Example | Heating temperature (°C) | Heating time (hr) | Thermal resistance (%) | Acid resistance (%) | Shear resistance (%) | Retort resistance (%) | Electrical conductivity (μS/cm) |
|---|---|---|---|---|---|---|---|
| 1 | 120 | 20 | 90 | 58 | 54 | 39 | 187 |
| 2 | 120 | 40 | 93 | 67 | 97 | 64 | 190 |
| 3 | 150 | 4 | 121 | 85 | 84 | 73 | 220 |
| 4 | 180 | 1 | 110 | 87 | 94 | 77 | 200 |
| 5 | 180 | 4 | 407 | 379 | 34 | 1708 | 290 |
| 6 | 200 | 1 | 163 | 160 | 58 | 335 | 270 |
| Comparative Example | Heating temperature (°C) | Heating time (hr) | Thermal resistance (%) | Acid resistance (%) | Shear resistance (%) | Retort resistance (%) | Electrical conductivity (μS/cm) |
| 1 | - | - | 73 | 14 | 15 | 7 | 147 |

(Example G: Relationship between amount of moisture and property of suppressed swelling)

(Experimental approach)

Resistant starches were prepared using ume juice as an acid containing preparation and waxy corn starch as a raw material starch in accordance with Example A. The method of preparing a resistant starch is the same as the method described in Example A, except for adjusting the amount of moisture prior to the reaction of raw material starch to 12.5%, 18.4%, 23.0%, or 34.7%, before the step of heating. Subsequently, thermal resistance, acid resistance, shear resistance, retort resistance, and electrical conductivity of the prepared resistant starches were measured.

(Results)

Table G shows the results of Example G. The resistant starches prepared by adjusting the amount of moisture prior to a reaction to 23.0% were demonstrated to exhibit excellent thermal resistance, acid resistance, shear resistance, and retort resistance and exhibit an excellent property of suppressed swelling. The resistant starches also had a low electrical conductivity and excellent flavor. Meanwhile, the thermal resistance, acid resistance, shear resistance, and retort resistance of the starch prepared by adjusting the amount of moisture prior to a reaction to 12.5% were lower than those of the resistant starches prepared by adjusting the amount of moisture to 18.4%, 23.0%, or 34.7%.

[Table G]

Table G. Difference in the amounts of moisture of moist starches

| Example | Amount of moisture prior to reaction (%) | Thermal resistance (%) | Acid resistance (%) | Shear resistance (%) | Retort resistance (%) | Electrical conductivity ($\mu$S/cm) |
|---|---|---|---|---|---|---|
| 1 | 18.4 | 95 | 70 | 37 | 30 | 200 |
| 2 | 23.0 | 121 | 85 | 84 | 73 | 220 |
| 3 | 34.7 | 112 | 85 | 103 | 78 | 160 |

| Comparative Example | Amount of moisture prior to reaction (%) | Thermal resistance (%) | Acid resistance (%) | Shear resistance (%) | Retort resistance (%) | Electrical conductivity ($\mu$S/cm) |
|---|---|---|---|---|---|---|
| 1 | 12.5 | 84 | 51 | 22 | 16 | 220 |

(Example H: Relationship between amount of acid containing preparation added and property of suppressed swelling)

(Experimental approach)

Resistant starches were prepared using ume juice as the acid containing preparation and waxy corn starch as the raw material starch in accordance with Example A. The method of preparing a resistant starch is the same as the method described in Example A, except for changing the amount of ume juice to be mixed with a raw material starch to 0.2%, 0.5%, 2.0%, 5.0%, 20%, or 50% with respect to the mass of the raw material starch. Subsequently, thermal resistance, acid resistance, shear resistance, retort resistance, and electrical conductivity of the prepared resistant starches and raw material starch were measured.

(Results)

Table H shows the results of Example H. The resistant starches prepared by adding ume juice at 2.0%, 5.0%, 20%, or 50% were demonstrated to exhibit excellent thermal resistance, acid resistance, shear resistance, and retort resistance and exhibit an excellent property of suppressed swelling. The resistant starches also had a low electrical conductivity and excellent flavor. While data is not shown, the resistant starch prepared by adding ume juice at 0.2% also had improved acid resistance and shear resistance and had a property of suppressed swelling.

[Table H]

**Table H. Difference in the amount of juice added**

| Example | Amount of juice added (%) | Acid content therein (%) | Acid resistance (%) | Shear resistance (%) | Retort resistance (%) | Electrical conductivity ($\mu$S/cm) |
|---|---|---|---|---|---|---|
| 1 | 0.5 | 0.022 | 63 | 31 | 40 | 71 |
| 2 | 2.0 | 0.088 | 75 | 64 | 47 | 136 |
| 3 | 5.0 | 0.220 | 85 | 84 | 73 | 147 |
| 4 | 20 | 0.880 | 97 | 99 | 141 | 310 |
| 5 | 50 | 2.200 | 101 | 89 | 163 | 320 |
| | | | | | | |
| Comparative Example | Amount of juice added (%) | Acid content therein (%) | Acid resistance (%) | Shear resistance (%) | Retort resistance (%) | Electrical conductivity ($\mu$S/cm) |
| 1 | Raw material waxy corn starch | - | 14 | 15 | 7 | 147 |

(Example I: Relationship between amount of organic acid, saccharide, and amino acid and property of suppressed swelling)

(Experimental approach)

This Example studied the relationship between the primary components of juice, i.e., organic acid, saccharide, and amino acid, and the property of suppressed swelling of starch. Acid containing preparations were prepared by using fructose (Example 1), asparagine (Asn) (Example 2), citric acid and malic acid (Example 3), fructose and Asn (Example 4), citric acid, malic acid, and fructose (Example 5), citric acid, malic acid, and Asn (Example 6), and citric acid, malic acid, fructose, and Asn (Example 7). The organic acid was added so that the amount would be 0.739% (corresponding to 16.8% ume juice), 0.220% (corresponding to 5% ume juice), 0.025% (corresponding to 0.57% ume juice), or 0.0220% (corresponding to 0.5% ume juice). Acid containing preparations adjusted so that the amount of organic acid added would be 0.220% (corresponding to 5% ume juice) were also prepared using fructose (Example 1), Asn (Example 2), citric acid alone (Example 3), fructose and Asn (Example 4), citric acid and fructose (Example 5), citric acid and Asn (Example 6), and citric acid, fructose, and Asn (Example 7). The amounts of saccharide (fructose) and amino acid (Asn) are described in the table. Resistant starches were prepared using these acid containing preparations and waxy corn starch as a raw material starch in accordance with Example A. Subsequently, retort resistance and electrical conductivity of the prepared resistant starches and raw material starch were measured.

(Results)

Table I shows the results of Example I. The resistant starches prepared by adding an organic acid to be 0.739% by adding citric acid, malic acid, and Asn, or citric acid, malic acid, fructose, and Asn were demonstrated to exhibit excellent retort resistance and an excellent property of suppressed swelling. Example 3 (using only organic acid as an acid containing preparation) also had a low electrical conductivity and excellent flavor. Meanwhile, the raw material starch had poor retort resistance. The resistant starch prepared using an acid containing preparation added with only citric acid as an organic acid also exhibited excellent retort resistance and low electrical conductivity under conditions of only citric acid, citric acid and fructose, citric acid and Asn, and citric acid, fructose, and Asn. The resistant starches prepared using an acid containing preparation comprising only fructose, only Asn, and fructose and Asn had the same degree of retort resistance as the raw material starch, so that retort resistance was not imparted.

[Table I-1]

**Table I-1. Effect of acid, saccharide, and amino acid**

| (0.739% acid + 0.084% saccharide + 0.067% amino acid were added (corresponding to 16.8% ume juice)) | | | |
|---|---|---|---|
| **Example** | | **Retort resistance (%)** | **Electrical conductivity ($\mu$S/cm)** |
| 1 | Fructose | 5 | 54 |
| 2 | Asn | 6 | 57 |
| 3 | Citric acid + malic acid | 34 | 290 |
| 4 | Fructose + Asn | 5 | 59 |
| 5 | Citric acid + malic acid + fructose | 47 | 300 |
| 6 | Citric acid + malic acid + Asn | 62 | 300 |
| 7 | Citric acid + malic acid + fructose + Asn | 70 | 310 |
| | | | |
| **Comparative Example** | | **Retort resistance (%)** | **Electrical conductivity ($\mu$S/cm)** |
| 1 | Raw material waxy corn starch | 7 | 147 |

[Table I-2]

**Table I-2. Effect of acid, saccharide, and amino acid**

| (0.22% acid + 0.025% saccharide + 0.020% amino acid were added (corresponding to 5% ume juice)) | | | |
|---|---|---|---|
| **Example** | | **Retort resistance (%)** | **Electrical conductivity ($\mu$S/cm)** |
| 1 | Fructose | 7 | 54 |
| 2 | Asn | 7 | 52 |
| 3 | Citric acid + malic acid | 32 | 200 |
| 4 | Fructose + Asn | 6 | 54 |
| 5 | Citric acid + malic acid + fructose | 40 | 195 |
| 6 | Citric acid + malic acid + Asn | 52 | 179 |
| 7 | Citric acid + malic acid + fructose + Asn | 51 | 191 |
| | | | |
| **Comparative Example** | | **Retort resistance (%)** | **Electrical conductivity ($\mu$S/cm)** |
| 1 | Raw material waxy corn starch | 7 | 147 |

[Table I-3]

**Table I-3. Effect of acid, saccharide, and amino acid**

| (0.025% acid + 0.0028% saccharide + 0.0023% amino acid were added (corresponding to 0.57% ume juice)) | | | |
|---|---|---|---|
| Example | | Retort resistance (%) | Electrical conductivity (μS/cm) |
| 1 | Fructose | 6 | 50 |
| 2 | Asn | 6 | 51 |
| 3 | Citric acid + malic acid | 32 | 61 |
| 4 | Fructose + Asn | 5 | 48 |
| 5 | Citric acid + malic acid + fructose | 39 | 66 |
| 6 | Citric acid + malic acid + Asn | 39 | 70 |
| 7 | Citric acid + malic acid + fructose + Asn | 43 | 66 |
| | | | |
| Comparative Example | | Retort resistance (%) | Electrical conductivity (μS/cm) |
| 1 | Raw material waxy corn starch | 7 | 147 |

[Table I-4]

**Table I-4. Effect of acid, saccharide, and amino acid**

| (0.022% acid + 0.0025% saccharide + 0.0020% amino acid were added (corresponding to 0.5% ume juice)) | | | |
|---|---|---|---|
| Example | | Retort resistance (%) | Electrical conductivity (μS/cm) |
| 1 | Fructose | 6 | 46 |
| 2 | Asn | 5 | 46 |
| 3 | Citric acid + malic acid | 32 | 60 |
| 4 | Fructose + Asn | 5 | 46 |
| 5 | Citric acid + malic acid + fructose | 31 | 64 |
| 6 | Citric acid + malic acid + Asn | 30 | 68 |
| 7 | Citric acid + malic acid + fructose + Asn | 33 | 64 |
| | | | |
| Comparative Example | | Retort resistance (%) | Electrical conductivity (μS/cm) |
| 1 | Raw material waxy corn starch | 7 | 147 |

[Table I-5]

**Table I-5. Effect of acid, saccharide, and amino acid (acid is only citric acid)**

| (0.22% acid + 0.025% saccharide + 0.020% amino acid were added (corresponding to 5% ume juice)) | | | |
|---|---|---|---|
| Example | | Retort resistance (%) | Electrical conductivity ($\mu$S/cm) |
| 1 | Fructose | 7 | 54 |
| 2 | Asn | 7 | 52 |
| 3 | Citric acid | 52 | 192 |
| 4 | Fructose + Asn | 6 | 54 |
| 5 | Citric acid + fructose | 68 | 198 |
| 6 | Citric acid + Asn | 72 | 200 |
| 7 | Citric acid + fructose + Asn | 89 | 210 |
| | | | |
| Comparative Example | | Retort resistance (%) | Electrical conductivity ($\mu$S/cm) |
| 1 | Raw material waxy corn starch | 7 | 147 |

(Example J: Relationship between type of organic acid and property of suppressed swelling)

(Experimental approach)

This Example studied the relationship between the type of organic acid and the property of suppressed swelling of starch. Acid containing preparations were prepared by using citric acid (Example 1), malic acid (Example 2), tartaric acid (Example 3), and quinic acid (Example 4). The organic acid added was so that the amount would be 0.165%. Resistant starches were prepared using these acid containing preparations and waxy corn starch as a raw material starch in accordance with Example A. Subsequently, retort resistance and electrical conductivity of the prepared resistant starches and raw material starch were measured.

(Results)

Table J shows the results of Example J. The resistant starches prepared by using citric acid, malic acid, and tartaric acid exhibited the same degree of retort resistance. The resistant starches also had a low electrical conductivity and excellent flavor. The resistant starch prepared by using quinic acid exhibited excellent electrical conductivity but low retort resistance. Meanwhile, the raw material starch had poor retort resistance.

[Table J]

**Table J. Comparison of acids for each acid alone**

| (0.165% acid added (corresponding to 5% ume juice)) | | | |
|---|---|---|---|
| Example | Type of acid | Retort resistance (%) | Electrical conductivity ($\mu$S/cm) |
| 1 | Citric acid | 30 | 153 |
| 2 | Malic acid | 29 | 178 |
| 3 | Tartaric acid | 21 | 170 |
| 4 | Quinic acid | 6 | 81 |
| | | | |
| Comparative Example | | Retort resistance (%) | Electrical conductivity ($\mu$S/cm) |
| 1 | Raw material waxy corn starch | 7 | 147 |

(Example K: Relationship between type of acid and property of suppressed swelling)

(Experimental approach)

This Example studied the relationship between the type of acid and the property of suppressed swelling of starch. Acid containing preparations were prepared by using citric acid (Example 1) and asconitic acid (Example 2) as tricarboxylic acid, malic acid (Example 3), tartaric acid (Example 4), oxalic acid (Example 5), and succinic acid (Example 6) as dicarboxylic acid, acetic acid (Example 7) and quinic acid (Example 8) as monocarboxylic acid, methanesulfonic acid (Example 9) and taurine (Example 10) as sulfonic acid, and ascorbic acid (Example 11), gluconic acid (Example 12), and glucuronic acid (Example 13) as sugar acid, and further using a mixture of citric acid and malic acid (Example 14) and mixture of citric acid and ascorbic acid (Example 15) as systems added with a plurality of types of organic acids. The organic acid was added to be 0.22%. As Comparative Examples, acid containing preparations were prepared using inorganic acids, i.e., hydrochloric acid and sulfuric acid. Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table K shows the results of Example K. The resistant starches prepared by using tricarboxylic acid, dicarboxylic acid, monocarboxylic acid, sugar acid, and mixture of a plurality of types of organic acids exhibited excellent retort resistance. The resistant starch prepared using sulfonic acid was also imparted with retort resistance. The resistant starches also had a low electrical conductivity and excellent flavor. Meanwhile, the resistant starches prepared by using an inorganic acid had poor retort resistance.

[Table K]

Table K. Comparison of acids for acid + saccharide + amino acid (0.22% acid + 0.025% saccharide + 0.020% amino acid were added (corresponding to 5% ume juice))

| Example | Type of acid | Retort resistance (%) | Electrical conductivity (μS/cm) |
|---|---|---|---|
| | <Tricarboxylic acid> | | |
| 1 | Citric acid | 89 | 210 |
| 2 | Asconitic acid | 74 | 165 |
| | <Dicarboxylic acid> | | |
| 3 | Malic acid | 83 | 200 |
| 4 | Tartaric acid | 43 | 189 |
| 5 | Oxalic acid | 45 | 179 |
| 6 | Succinic acid | 60 | 161 |
| | <Monocarboxylic acid> | | |
| 7 | Acetic acid | 58 | 210 |
| 8 | Quinic acid | 56 | 120 |
| | <Sulfonic acid> | | |
| 9 | Methansulfonic acid | 18 | 190 |
| 10 | Taurine | 16 | 120 |
| | <Sugar acid> | | |
| 11 | Ascorbic acid | 40 | 192 |
| 12 | Gluconic acid | 44 | 162 |

(continued)

| Example | Type of acid | Retort resistance (%) | Electrical conductivity (μS/cm) |
|---|---|---|---|
| 13 | Glucuronic acid | 64 | 159 |
| | <System with multiple additions> | | |
| 14 | 0.17% citric acid + 0.06% malic acid | 51 | 191 |
| 15 | 0.11% citric acid + 0.11% ascorbic acid | 64 | 194 |

| Comparative Example | | Retort resistance (%) | Electrical conductivity (μS/cm) |
|---|---|---|---|
| 1 | Raw material waxy | 7 | 147 |
| | corn starch | | |
| | <Inorganic acid> | | |
| 2 | Hydrochloric acid | 3 | 210 |
| 3 | Sulfuric acid | 2 | 200 |

(Example L: Relationship between type of saccharide and property of suppressed swelling)

(Experimental approach)

This Example studied the relationship between the type of saccharide and the property of suppressed swelling of starch. Acid containing preparations were prepared by using a mixture of 0.554% citric acid and 0.185% malic acid with sucrose (Example 1), glucose (Example 2), or fructose (Example 3), and a saccharide free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table L shows the results of Example L. The resistant starches prepared by using an acid containing preparation comprising glucose as a saccharide exhibited excellent retort resistance. The resistant starches also had a low electrical conductivity and excellent flavor. Meanwhile, the raw material starch had poor retort resistance.

[Table L]

**Table L. Comparison of saccharides for acid + saccharide**

| (0.739% acid + 0.084% saccharide were added (corresponding to 16.8% ume juice)) | | | |
|---|---|---|---|
| Example | Type of acid | Retort resistance (%) | Electrical conductivity (μS/cm) |
| 1 | Sucrose | 41 | 300 |
| 2 | Glucose | 58 | 300 |
| 3 | Fructose | 47 | 300 |

(continued)

| Comparative Example | | Retort resistance (%) | Electrical conductivity (μS/cm) |
|---|---|---|---|
| 1 | Raw material waxy corn starch | 7 | 147 |
| 2 | Citric acid + malic acid (no saccharide) | 34 | 290 |

(Example M: Relationship between type of saccharide added in the presence of acid and property of suppressed swelling)

(Experimental approach)

This Example studied the relationship between the type of saccharide added in the presence of citric acid and the property of suppressed swelling of saccharide. Acid containing preparations were prepared by using a mixture of 0.739% citric acid with sucrose (Example 1), glucose (Example 2), or fructose (Example 3), and a saccharide free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table M shows the results of Example M. The prepared resistant starches exhibited excellent retort resistance, with an acid containing preparation prepared using any of the saccharides in combination with citric acid. The retort resistance thereof was higher than retort resistance from using citric acid alone. The resistant starches also had a low electrical conductivity and excellent flavor. Meanwhile, the raw material starch had poor retort resistance.

[Table M]

**Table M. Comparison of saccharides for acid + saccharide**

| (0.739% acid + 0.084% saccharide were added (corresponding to 16.8% ume juice)) | | | |
|---|---|---|---|
| **Example** | | Retort resistance (%) | Electrical conductivity (μS/cm) |
| 1 | Sucrose | 58 | 300 |
| 2 | Glucose | 65 | 310 |
| 3 | Fructose | 62 | 300 |
| | | | |
| **Comparative Example** | | Retort resistance (%) | Electrical conductivity (μS/cm) |
| 1 | Raw material waxy corn starch | 7 | 147 |
| 2 | Citric acid only (no saccharide) | 42 | 290 |

(Example N: Relationship between type of saccharide added in the presence of acid and amino acid and property of suppressed swelling)

(Experimental approach)

This Example studied the relationship between the type of saccharide added in the presence of citric acid, malic acid, and amino acid and the property of suppressed swelling of starch. Acid containing preparations were prepared by using a mixture of 0.554% citric acid, 0.185% malic acid, and 0.067% amino acid (asparagine) with sucrose (Example 1), glucose (Example 2), or fructose (Example 3), and saccharide free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table N shows the results of Example N. The prepared resistant starches exhibited excellent retort resistance, with an acid containing preparation prepared using any of the saccharides in combination with citric acid, malic acid, and amino acid. The retort resistance thereof was higher than retort resistance from using only citric acid and amino acid. The resistant starches also had a low electrical conductivity and excellent flavor. Meanwhile, the raw material starch had poor retort resistance.

[Table N]

**Table N. Comparison of saccharides for acid + saccharide + amino acid**

| (0.739% acid + 0.084% saccharide + 0.067% amino acid were added (corresponding to 16.8% ume juice)) | | | |
|---|---|---|---|
| Example | Type of acid | Retort resistance (%) | Electrical conductivity ($\mu$S/cm) |
| 1 | Sucrose | 67 | 310 |
| 2 | Glucose | 72 | 300 |
| 3 | Fructose | 70 | 300 |
| | | | |
| Comparative Example | Type of acid | Retort resistance (%) | Electrical conductivity ($\mu$S/cm) |
| 1 | Raw material waxy corn starch | 7 | 147 |
| 2 | Citric acid + malic acid + amino acid (no saccharide) | 62 | 300 |

(Example O : Relationship between type of amino acid and property of suppressed swelling)

(Experimental approach)

This Example studied the relationship between the type of amino acid and the property of suppressed swelling of starch. Acid containing preparations were prepared by using a mixture of 0.165% citric acid and 0.055% malic acid with Asn (Example 1), Gln (Example 2), Glu (Example 3), Arg (Example 4), Ser (Example 5), Trp (Example 6), Cys (Example 7), Ala (Example 8), or Pro (Example 9), and amino acid free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table O shows the results of Example O. The resistant starches prepared using an acid containing preparation comprising Asn or Cys as an amino acid exhibited excellent retort resistance. The resistant starches also had a low electrical conductivity and excellent flavor. The result suggested the possibility of usefulness of neutral amino acid in the manufacture of resistant starches. Meanwhile, the raw material starch had poor retort resistance.

[Table O]

Table O. Comparison of amino acids for acid + amino acid

| (0.22% acid + 0.020% amino acid were added (corresponding to 5% ume juice)) | | | |
|---|---|---|---|
| Example | Type of amino acid | Retort resistance (%) | Electrical conductivity (μS/cm) |
| 1 | Asn | 52 | 179 |
| 2 | Gln | 42 | 194 |
| 3 | Glu | 37 | 210 |
| 4 | Arg | 36 | 200 |
| 5 | Ser | 47 | 190 |
| 6 | Trp | 41 | 184 |
| 7 | Cys | 55 | 160 |
| 8 | Ala | 42 | 165 |
| 9 | Pro | 39 | 200 |
| | | | |
| Comparative Example | | Retort resistance (%) | Electrical conductivity (μS/cm) |
| 1 | Raw material waxy corn starch | 7 | 147 |
| 2 | Citric acid + malic acid (no amino acid) | 32 | 200 |

(Example P: Relationship between type of amino acid in the presence of acid and property of suppressed swelling)

(Experimental approach)

This Example studied the relationship between the type of amino acid added in the presence of acid and the property of suppressed swelling of starch. Acid containing preparations were prepared by using a mixture of 0.22% citric acid with Asn (Example 1), Gln (Example 2), Glu (Example 3), Arg (Example 4), Ser (Example 5), Trp (Example 6), Cys (Example 7), Ala (Example 8), or Pro (Example 9), and amino acid free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table P shows the results of Example P. The prepared resistant starches exhibited excellent retort resistance, with an acid containing preparation prepared using any of the amino acids in combination with citric acid. The retort resistance thereof was higher than retort resistance from using citric acid alone. The resistant starches also had a low electrical conductivity and excellent flavor. Meanwhile, the raw material starch had poor retort resistance.

[Table P]

Table P. Comparison of amino acids for acid + amino acid

| (0.22% acid + 0.020% amino acid were added (corresponding to 5% ume juice)) | | | |
|---|---|---|---|
| Example | | Retort resistance (%) | Electrical conductivity (μS/cm) |
| 1 | Asn | 72 | 210 |
| 2 | Gln | 61 | 195 |

(continued)

| (0.22% acid + 0.020% amino acid were added (corresponding to 5% ume juice)) | | | |
|---|---|---|---|
| Example | | Retort resistance (%) | Electrical conductivity (μS/cm) |
| 3 | Glu | 58 | 210 |
| 4 | Arg | 54 | 200 |
| 5 | Ser | 65 | 191 |
| 6 | Trp | 61 | 185 |
| 7 | Cys | 73 | 170 |
| 8 | Ala | 62 | 175 |
| 9 | Pro | 58 | 200 |
| | | | |
| Comparative Example | | Retort resistance (%) | Electrical conductivity (μS/cm) |
| 1 | Raw material waxy corn starch | 7 | 147 |
| 2 | Citric acid only (no amino acid) | 52 | 180 |

(Example Q: Relationship between type of amino acid in the presence of acid and saccharide and property of suppressed swelling)

(Experimental approach)

This Example studied the relationship between the type of amino acid added in the presence of acid and saccharide and the property of suppressed swelling of starch. Acid containing preparations were prepared by using a mixture of 0.165% citric acid, 0.055% malic acid, and 0.025% saccharide (fructose) with Asn (Example 1), Gln (Example 2), Glu (Example 3), Arg (Example 4), Ser (Example 5), Trp (Example 6), Cys (Example 7), Ala (Example 8), or Pro (Example 9), and amino acid free mixture (Comparative Example 2). Resistant starches were prepared using these acid containing preparations and waxy corn starch as the raw material starch in accordance with Example A. Subsequently, retort resistance and electrical conductivity of the prepared resistant starches and raw material starch were measured.

(Results)

Table Q shows the results of Example Q. The prepared resistant starches exhibited excellent retort resistance, with an acid containing preparation prepared using any of the amino acids in combination with acid and saccharide. The retort resistance thereof was higher than retort resistance from using only acid and saccharide, except for Arg (Example 4). The resistant starches also had a low electrical conductivity and excellent flavor. Meanwhile, the raw material starch had poor retort resistance.

[Table Q]

Table Q. Comparison of amino acids for acid + saccharide + amino acid

| (0.22% acid + 0.025% saccharide + 0.020% amino acid were added (corresponding to 5% ume juice)) | | | |
|---|---|---|---|
| Example | Type of amino acid | Retort resistance (%) | Electrical conductivity (μS/cm) |
| 1 | Asn | 51 | 191 |
| 2 | Gln | 46 | 199 |
| 3 | Glu | 41 | 220 |
| 4 | Arg | 40 | 210 |

(continued)

| (0.22% acid + 0.025% saccharide + 0.020% amino acid were added (corresponding to 5% ume juice)) | | | |
|---|---|---|---|
| Example | Type of amino acid | Retort resistance (%) | Electrical conductivity ($\mu$S/cm) |
| 5 | Ser | 51 | 195 |
| 6 | Trp | 43 | 190 |
| 7 | Cys | 56 | 171 |
| 8 | Ala | 46 | 178 |
| 9 | Pro | 41 | 210 |

| Comparative Example | | Retort resistance (%) | Electrical conductivity ($\mu$S/cm) |
|---|---|---|---|
| 1 | Raw material waxy corn starch | 7 | 147 |
| 2 | Citric acid + malic acid + saccharide (no amino acid) | 40 | 195 |

(Example R: Property of suppressed swelling when the amounts of acid, saccharide, and amino acid are varied)

(Experimental approach)

This Example compared the property of suppressed swelling of starch prepared by varying the amounts of acid, saccharide, and amino acid. Acid containing preparations were prepared by varying the amount of citric acid to 0.022%, 0.22% and 3.45%, the amount of fructose to 0.0025%, 0.025%, and 1.6%, and the amount of Asn to 0.0015%, 0.02%, and 0.2%. Resistant starches were prepared using these acid containing preparations and waxy corn starch as the raw material starch in accordance with Example A. As a control, a raw material waxy corn starch was used. Subsequently, retort resistance and electrical conductivity of the prepared resistant starches and raw material starch were measured.

(Results)

Table R shows the results of Example R. The prepared resistant starches exhibited excellent retort resistance, with a combination of citric acid, fructose, and Asn at any of the amounts. The resistant starches also had a low electrical conductivity and excellent flavor. Meanwhile, the raw material starch had poor retort resistance.

[Table R]

#### Table R. Amounts of acid/saccharide/amino acid were varied

| Example | | Citric acid (%) | Fructose (%) | Asn (%) | Retort resistance (%) | Electrical conductivity ($\mu$S/cm) |
|---|---|---|---|---|---|---|
| 2 | | 0.022 | 0.025 | 0.02 | 52 | 81 |
| 1 | | 0.22 | 0.025 | 0.02 | 89 | 210 |
| 3 | | 3.45 | 0.025 | 0.02 | 58 | 300 |
| 4 | | 0.22 | 0.0025 | 0.02 | 85 | 200 |
| 1 | | 0.22 | 0.025 | 0.02 | 89 | 210 |
| 5 | | 0.22 | 0.25 | 0.02 | 195 | 240 |

(continued)

| Example | | Citric acid (%) | Fructose (%) | Asn (%) | Retort resistance (%) | Electrical conductivity (μS/cm) |
|---|---|---|---|---|---|---|
| 6 | | 0.22 | 1.6 | 0.02 | 391 | 260 |
| 7 | | 0.22 | 0.025 | 0.0015 | 72 | 200 |
| 1 | | 0.22 | 0.025 | 0.02 | 89 | 210 |
| 8 | | 0.22 | 0.025 | 0.2 | 136 | 240 |
| 9 | Two were varied | 0.022 | 1.6 | 0.02 | 160 | 187 |
| 10 | | 0.22 | 0.0025 | 0.2 | 50 | 240 |
| 11 | | 3.45 | 0.025 | 0.0015 | 55 | 300 |
| 12 | Three were varied | 0.022 | 1.6 | 0.0015 | 46 | 185 |
| 13 | | 0.022 | 0.0025 | 0.2 | 45 | 152 |
| 14 | | 3.45 | 0.0025 | 0.0015 | 54 | 300 |
| 15 | | 0.022 | 0.0025 | 0.0015 | 56 | 62 |
| 16 | | 3.45 | 1.6 | 0.2 | 80 | 310 |

| Comparative Example | | Citric acid (%) | Fructose (%) | Asn (%) | Retort resistance (%) | Electrical conductivity (μS/cm) |
|---|---|---|---|---|---|---|
| 1 | Raw material waxy corn starch | - | - | - | 7 | 147 |

(Example S: Property of suppressed swelling when the amounts of acid, saccharide, and amino acid are varied)

(Experimental approach)

This Example compared the properties of suppressed swelling of starches prepared by including acid but without including either saccharide or amino acid and varying the amounts of acid and saccharide or amino acid. Acid containing preparations were prepared by varying the amount of citric acid to 0.022%, 0.22%, and 3.45%, the amount of fructose to 0%, 0.0025%, 0.025%, and 1.6%, and the amount of Asn to 0%, 0.0015%, 0.02%, and 0.2%. Resistant starches were prepared using these acid containing preparations and waxy corn starch as the raw material starch in accordance with Example A. As a control, a raw material waxy corn starch was used. Subsequently, retort resistance and electrical conductivity of the prepared resistant starches and raw material starch were measured.

(Results)

Table S shows the results of Example S. The prepared resistant starches exhibited excellent retort resistance under any of the conditions in this Example. The resistant starches also had a low electrical conductivity and excellent flavor. Meanwhile, the raw material starch had poor retort resistance.

[Table S]

**Table S. Amounts of acid/saccharide/amino acid were varied (saccharide or amino acid added system)**

| | Example | Citric acid (%) | Fructose (%) | Asn (%) | Retort resistance (%) | Electrical conductivity (μS/cm) |
|---|---|---|---|---|---|---|
| 1 | | 0.022 | 0.025 | - | 53 | 80 |
| 2 | | 0.22 | 0.025 | - | 68 | 198 |
| 3 | | 3.45 | 0.025 | - | 52 | 280 |
| 4 | | 0.22 | 0.0025 | - | 56 | 192 |
| 5 | | 0.22 | 0.25 | - | 180 | 210 |
| 6 | | 0.22 | 1.6 | - | 360 | 240 |
| 7 | | 0.022 | - | 0.02 | 45 | 157 |
| 8 | | 0.22 | - | 0.02 | 72 | 200 |
| 9 | | 3.45 | - | 0.02 | 53 | 300 |
| 10 | | 0.22 | - | 0.0015 | 51 | 210 |
| 11 | | 0.22 | - | 0.2 | 47 | 240 |
| 12 | Two were varied | 0.022 | 0.0025 | - | 48 | 61 |
| 13 | | 3.45 | 1.6 | - | 70 | 290 |
| 14 | | 0.022 | - | 0.0015 | 49 | 60 |
| 15 | | 3.45 | - | 0.2 | 60 | 290 |
| | | | | | | |
| | Comparative Example | Citric acid (%) | Fructose (%) | Asn (%) | Retort resistance (%) | Electrical conductivity (μS/cm) |
| 1 | Raw material waxy corn starch | - | - | - | 7 | 147 |

(Example T: Comparison of presence/absence of washing and property of suppressed swelling)

(Experimental approach)

This Example compared the properties of suppressed swelling between methods of manufacturing the resistant starch of the present disclosure described in Example A with or without washing. Resistant starches were prepared by mixing an acid containing preparation that is one of (1) 5.0% ume juice, (2) mixture of fructose, asparagine, citric acid, and malic acid corresponding to 5.0% ume juice, and (3) 5.0% lemon juice with a raw material starch, i.e., waxy corn starch or tapioca starch, and washing or without washing with water. Subsequently, electrical conductivity, flavor, and scent of the manufactured resistant starches were measured.

For the electrical conductivity, the supernatant was measured after adding 3.0 g of starch in dry basis to ultrapure water so that the total would be 9.0 g (33% in dry basis) and centrifuging the mixture for 10 minutes at 2000 × g. The temperature of 30 g of 5% by mass starch slurry was increased over 13 minutes from 35°C to 95°C, maintained for 10 minutes, and then cooled over 14 minutes to 25°C with a Rapid Visco Analyzer (Perten Instruments). The flavor and scent of the produced starch solution were evaluated by a four member panel. The starch was scored from 1 to 5 in a 5 point evaluation system (higher evaluation for higher scores). As a comparison, a product of another manufacturer (Novation 2600) was also evaluated.

(Results)

Table T shows the results of Example T. Each of the waxy corn starch treated with 5.0% ume juice or a mixture of fructose, asparagine, citric acid, and malic acid corresponding to 5.0% ume juice, and tapioca starch treated with 5.0% lemon juice had electrical conductivity of 1000 or greater without washing. Unwashed starches had flavor and scent evaluation of 1 to 3, with a strong burnt taste and scent understood to be due to heating. Meanwhile, each of the washed starches had electrical conductivity of 240 or less, flavor and scent evaluation of 4, and better flavor and scent than unwashed products. Furthermore, each of the washed starches had better electrical conductivity, flavor, and scent than Novation 2600.

[Table T]

**Table T. Evaluation of retort resistance, electrical conductivity, and starch solution flavor in the presence/ absence of washing**

| Samples | | Retort resistance (%) | Electrical conductivity ($\mu$S/cm) | Flavor | Scent |
|---|---|---|---|---|---|
| Waxy cornstarch added with 5.0% ume juice | Unwashed product | 72 | 1080 | 1 | 1 |
| | Washed product | 73 | 220 | 4 | 4 |
| added with acid/saccharide/amino acid corresponding to 5.0% ume juice | Unwashed product | 50 | 1140 | 3 | 3 |
| | Washed product | 51 | 191 | 4 | 4 |
| Tapioca added with 5.0% lemon juice | Unwashed product | 78 | 1840 | 1 | 1 |
| | Washed product | 80 | 240 | 4 | 4 |
| (Reference) Novation 2600 | | 49 | 350 | 2 | 2 |

(Example U: Comparison with products of other manufacturers)

(Experimental approach)

This Example compared the properties of suppressed swelling between the resistant starch of the present disclosure and swelling suppressed starches of other manufacturers. The resistant starch of the present disclosure was manufactured in accordance with the method described in Example A by using waxy corn starch and tapioca starch as raw material starches and ume juice as an acid containing preparation. Novation® 2300, 2600, 2700, and 3300, and Prima 600 (Ingredion) and Claria® Plus and Elite (Tate&Lyle) were used as swelling suppressed starches of other manufacturers. Subsequently, thermal resistance, acid resistance, shear resistance, retort resistance, and electrical conductivity of the manufactured resistant starch, swelling suppressed starches of other manufacturers, and raw material starches were measured.

(Results)

Table U shows the results of Example U. The resistant starch of the present disclosure exhibited excellent values in each of thermal resistance, acid resistance, shear resistance, retort resistance, and electrical conductivity. Meanwhile, the swelling suppressed starches of other manufacturers had high electrical conductivity and poor flavor. The raw material starches had poor thermal resistance, acid resistance, shear resistance, and retort resistance.

**[Table U]**

**Table U. Comparative with products of other manufacturers**

| Example | | Thermal resistance (%) | Acid resistance (%) | Shear resistance (%) | Retort resistance (%) | Electrical conductivity (μS/cm) |
|---|---|---|---|---|---|---|
| 1 | Resistant waxy corn starch | 121 | 85 | 84 | 73 | 220 |
| 2 | Resistant tapioca starch | 99 | 67 | 82 | 71 | 250 |

| Comparative Example | | Thermal resistance (%) | Acid resistance (%) | Shear resistance (%) | Retort resistance (%) | Electrical conductivity (μS/cm) |
|---|---|---|---|---|---|---|
| 1 | Raw material waxy corn starch | 73 | 14 | 15 | 7 | 147 |
| 2 | Novation 2300 | 102 | 84 | 93 | 73 | 390 |
| 3 | Novation 2600 | 94 | 72 | 76 | 49 | 350 |
| 4 | Novation 2700 | 86 | 58 | 53 | 32 | 410 |
| 5 | Novation Prima 600 | 89 | 62 | 64 | 54 | 480 |
| 6 | Claria Plus | - | - | - | 113 | 850 |
| 7 | Claria Elite | - | - | - | 120 | 860 |
| 8 | Raw material tapioca starch | 82 | 15 | 8 | 4 | 430 |
| 9 | Novation 3300 | 113 | 99 | 105 | 176 | 470 |

(Example V: Sensory evaluation)

Sensory evaluation was conducted on food products prepared using the resistant starch of the present disclosure. As a comparison with the resistant starch of the present disclosure, Novation® (Ingredion; physically processed starch of another manufacturer) and Colflo® 67 (Ingredion; modified starch) were used. The sensory evaluation was conducted by a four member panel. Mango sauce and Mitarashi sauce were evaluated on the quality of flavor, and grilled chicken sauce was evaluated on the quality of flavor as well as aftertaste from glazing chicken meat with the sauce and consuming the chicken. The flavor and aftertaste were scored in a 5 point evaluation system (higher evaluation for higher scores).

(Example W: Sensory evaluation of mango sauce)

(Experimental approach)

This Example shows sensory evaluation on mango sauce prepared by using the resistant starch of the present disclosure. The resistant starch was prepared using waxy corn starch as the raw material starch, and ume juice as the acid containing preparation. The mango sauce was prepared using the ingredients described in the following table.

[Table W-1]

Table W-1. Proportion of ingredients of mango sauce

|  | % |
|---|---|
| **Starch** | 35 |
| **Mango juice** | 40 |
| **Granulated sugar** | 23 |
| **Water** | 33.5 |

Liquid ingredients and premixed powder were added to a stainless steel beaker and heated to 60°C in a 95°C thermostatic vessel while mixing with a thermometer. After heating, the mixture was stirred with a double blade impeller while heating for 20 minutes at 750 rpm in the 95°C thermostatic vessel. The prepared mango sauce was cooled in ice water. The flavor of the prepared mango sauce was subjected to sensory evaluation.

(Results)

The results of Example W are shown in Table W-2. The mango sauce prepared by using the resistant starch of the present disclosure had a lower grain scent than the mango sauce prepared using the raw material starch. In addition, the mango sauce prepared by using the resistant starch of the present disclosure had a mild flavor with less bitterness compared to the physically processed starches of other manufacturers and modified starch.

[Table W-2]

Table W-2. Mango sauce

| Evaluated item | Raw material waxy corn starch | Resistant waxy corn starch | Novation 2600 | Colflo 67 |
|---|---|---|---|---|
| **Flavor** | 3 | 5 | 2 | 2 |

(Example W-3: Sensory evaluation on mango sauce)

(Experimental approach)

This Example shows sensory evaluation on mango sauce when a resistant starch was prepared by changing the juice used as an acid containing preparation. The resistant starch was prepared by using waxy corn starch as a raw material starch and lemon, lime, shikuwasa, orange, grapefruit, or apple juice as an acid containing preparation. The mango sauce was prepared in accordance with the cooking method and ingredients in the table described in Example W-1. The flavor of the prepared mango sauce was subjected to sensory evaluation.

(Results)

The results of Example W-3 are shown in Table W-3. The highest evaluation was given when using an acid containing preparation prepared using lemon juice. High evaluation was also given when using an acid containing preparation prepared using lime, shikuwasa, orange, grapefruit, and apple juice. In addition, sauce using an acid containing preparation prepared by using any of the juice had no bitterness found with the physically processed starch of other manufacturers and modified starch and had better flavor than the physically processed starch of other manufacturers and modified starch.

[Table W-3]

## Table W-3. Mango sauce (when type of juice was changed)

| Evaluated item | Lemon | Lime | Shikuwasa | Orange | Grapefruit | Apple |
|---|---|---|---|---|---|---|
| Flavor | 5 | 5 | 5 | 4 | 4 | 4 |

| Evaluated item | Novation 2600 | Colflo 67 |
|---|---|---|
| Flavor | 5 | 5 |

(Example X: Sensory evaluation on Mitarashi sauce)

(Experimental approach)

This Example shows sensory evaluation on Mitarashi sauce prepared by using the resistant starch of the present disclosure. The resistant starch was prepared using waxy corn starch as a raw material starch and ume juice as an acid containing preparation. The Mitarashi sauce was prepared using the ingredients described in the following table. The monosodium glutamate in the table was purchased from Ajinomoto Co., Inc.

[Table X-1]

### Table X-1. Proportion of ingredients of Mitarashi sauce

| | % |
|---|---|
| Starch | 4.0 |
| Granulated sugar | 29.8 |
| Soy sauce | 17.4 |
| Mirin | 5.0 |
| Monosodium glutamate | 0.03 |
| Water | 43.8 |

All ingredients in the table were added to a stainless steel beaker and heated to 60°C in a 95°C thermostatic vessel while mixing with a thermometer. After heating, the mixture was stirred while heating for 20 minutes at 750 rpm in the 95°C thermostatic vessel. The prepared Mitarashi sauce was cooled in ice water. The flavor of the prepared Mitarashi sauce was subjected to sensory evaluation.

(Results)

The results of Example X are shown in Table X-2. The Mitarashi sauce prepared by using the resistant starch of the present disclosure had a lower grain scent than the Mitarashi sauce prepared using the raw material starch, with a strong flavor from soy sauce. In addition, the mango sauce prepared by using the resistant starch of the present disclosure had less bitterness compared to sauce prepared using the physically processed starches of other manufacturers and modified starch, with a sensation of more markedly milder flavor.

[Table X-2]

**Table X-2. Mitarashi sauce**

| Evaluated item | Raw material waxy corn starch | Resistant waxy corn starch | Novation 2600 | Claria Elite | Colflo 67 |
|---|---|---|---|---|---|
| Flavor | 3 | 5 | 2 | 2 | 2 |

(Example Y: Sensory evaluation on grilled chicken sauce)

(Experimental approach)

This Example shows sensory evaluation on grilled chicken sauce prepared by using the resistant starch of the present disclosure. The resistant starch was prepared using waxy corn starch as a raw material starch and ume juice as an acid containing preparation. The grilled chicken sauce was prepared using the ingredients described in the following table.

[Table Y-1]

**Table Y-1. Proportion of ingredients of grilled chicken sauce**

|  | % |
|---|---|
| Starch | 4.5 |
| Caster sugar | 22 |
| Powdered kelp stock | 0.5 |
| Powdered katsuo stock | 0.3 |
| Ajinomoto monosodium glutamate | 0.7 |
| Cooking sake | 15 |
| Rice vinegar | 12 |
| Dark soy sauce | 30 |
| Water | 15 |

Liquid ingredients and premixed powder were added to a stainless steel beaker and heated to 60°C in a 95°C thermostatic vessel while mixing with a thermometer. After heating, the mixture was stirred with a propeller blade while heating for 20 minutes at 750 rpm in the 95°C thermostatic vessel. The prepared sauce was cooled in ice water.

Two types of sensory evaluations were conducted, i.e., grilled chicken sauce alone, and chicken meat glazed with grilled chicken sauce. Chicken meat glazed with grilled chicken sauce was prepared in the following manner.

1. Chicken breast meat was divided into 15 g pieces.
2. 3 g of salad oil was added to a frying pan, and 4 pieces of chicken meat were cooked for 5 minutes over an electrical stove (medium heat).
3. chicken meat was cooked for 1 minute while adding 70 g of grilled chicken sauce to glaze the chicken meat.
4. Sensory evaluation was conducted after allowing the chicken to cool down.

(Results)

The results of Example Y are shown in Table Y-2. The grilled chicken sauce prepared by using the resistant starch of the present disclosure, when subjected to a sensory evaluation alone, had less bitterness than the sauce prepared using the raw material starch, the physically processed starches of other manufacturers, or modified starch, with a sensation of more markedly milder flavor and hardly any grain scent. When grilled chicken was glazed with grilled chicken sauce, the grilled chicken sauce prepared using the resistant starch of the present disclosure had the same quality of flavor as the raw material starch and weaker flavor than those prepared using the physically processed starch of other manufacturers and modified starch, but was able to utilize the inherent flavor of the ingredients.

[Table Y-2]

**Table Y-2. Grilled chicken sauce**

| Evaluated item | Raw material waxy corn starch | Resistant waxy corn starch | Novation 2600 | Colflo 67 |
|---|---|---|---|---|
| **Flavor** | 3 | 4 | 1 | 1 |
| **Aftertaste (when chicken meat was glazed)** | 2 | 2 | 4 | 4 |

(Example Z: Sensory evaluation on custard)

(Experimental approach)

This Example shows sensory evaluation on custard prepared by using the resistant starch of the present disclosure. The resistant starch was prepared by using corn starch, waxy corn starch, glutinous rice starch, rice starch, and potato starch as raw material starches and using ume juice as an acid containing preparation. The custard was prepared by using the ingredients described in the following table. The oligotose in the table was obtained from Sanwa Starch Co., Ltd. Sensory evaluation was conducted by a four member panel. The sensory evaluation evaluated the quality of flavor and smoothness, which were scored by a 5 point evaluation system (higher evaluation for higher scores).

[Table Z-1]

**Table Z-1. Proportion of ingredients of custard**

| | % |
|---|---|
| **Starch** | 5 |
| **Granulated sugar** | 7.5 |
| **Milk** | 56.1 |
| **Oligotose** | 22 |
| **Egg yolk** | 7 |
| **Salt free margarine** | 2.4 |

(Results)

The results of Example Z are shown in Table Z-2. The custard prepared by using the resistant starch of the present disclosure, regardless of which raw material starch the custard was prepared from, had a lower grain scent than the custard prepared using the raw material starch. Off-taste or sever stickiness found with the physically processed starches of other manufacturers were not found in the custard prepared by using the resistant starch of the present disclosure.

[Table Z-2]

## Table Z-2. Custard

| Evaluated item | Raw material corn starch | Raw material waxy corn starch | Raw material glutinous rice starch | Raw material rice starch | Raw material potato starch | Novation 2300 |
|---|---|---|---|---|---|---|
| Flavor | 2 | 1 | 2 | 1 | 2 | 1 |
| Smoothness | 2 | 2 | 2 | 2 | 1 | 2 |

| Evaluated item | Resistant corn starch | Resistant waxy corn starch | Resistant glutinous rice starch | Resistant rice starch | Resistant potato starch |
|---|---|---|---|---|---|
| Flavor | 4 | 4 | 5 | 5 | 4 |
| Smoothness | 4 | 4 | 5 | 5 | 4 |
| pH prior to heating of starch | 4.20 | 6.19 | 4.78 | 5.12 | 5.27 |

(Example AA: Sensory evaluation on yogurt)

(Experimental approach)

This Example shows sensory evaluation on yogurt prepared by using the resistant starch of the present disclosure. The resistant starch was prepared using tapioca starch as a raw material starch, and ume juice and lemon juice as acid containing preparations. The yogurt was prepared using the ingredients described in the following table. As the starter in the table, Meiji Bulgaria Yogurt (plain) (Meiji Co., Ltd.) was used. Novation 3300 and C☆CreamTex 75720 (Cargill, modified starch) were used as subjects of comparison.

[Table AA-1]

**Table AA-1. Proportion of ingredients of yogurt**

|  | % |
|---|---|
| **Starch** | 1 |
| **Milk** | 87 |
| **Granulated sugar** | 8 |
| **Starter** | 4 |

The yogurt manufacturing process is the following.

1. Add milk, starch, and granulated sugar to a stainless steel beaker.
2. Heat and stir (200 rpm, 15 minutes, 65°C, single blade).
3. Homogenize (180 bar/50 bar, 1 pass).
4. Sterilize by heating (leave standing, 10 minutes, 95°C).
5. Cool with ice water until the temperature is 43°C.
6. Add starter at an amount that would be 4% of the entire amount and stir gently.
7. Allow fermentation (temperature: 42°C, humidity: 65%, 4 hours) and measure pH.
8. Cool with ice water, filter with a 60 mesh sieve, and mill the curd.
9. Fill a container and store in a refrigerator (4°C). Evaluate texture and flavor after 7 days.

Sensory evaluation was conducted by a four-member panel. The sensory evaluation evaluated the texture and quality of flavor, which were scored by a 5 point evaluation system (higher evaluation for higher scores)

(Results)

The results of Example AA are shown in Table AA-2. The yogurt prepared by using the resistant starch of the present disclosure, regardless of which juice was used to prepare the yogurt, reduced stickiness found in yogurt prepared by using the raw material starch and had smooth texture without coarseness. Improvement in texture was also found with Novation. Yogurt prepared by using the resistant starch of the present disclosure had reduced unevenness in aftertaste found in yogurt from modified starch, excellent flavor, and strong residual acerbic aftertaste.

[Table AA-2]

Table AA-2. Yogurt

| Evaluated item | Raw material tapioca starch | Resistant tapioca (ume juice) | Resistant tapioca (lemon juice) | Novation 3300 | C☆CreamTex 75720 |
|---|---|---|---|---|---|
| **Texture** | 3 | 4 | 4 | 4 | 3 |
| **Flavor** | 3 | 4 | 5 | 3 | 2 |

As disclosed above, the present disclosure is exemplified by the use of its preferred embodiments. However, the present disclosure should not be interpreted to be limited to such embodiments. It is understood that the scope of the present disclosure should be interpreted based solely on the Claims. It is understood that an equivalent scope can be practiced by those skilled in the art based on the descriptions of the present disclosure and common general knowledge from the specific descriptions in the preferred embodiments of the present disclosure. It is also understood that any patent, any patent application, and any references cited herein should be incorporated herein by reference in the same manner as the contents are specifically described herein. The present application claims priority to Japanese Patent Application No. 2018-206925 filed on November 1, 2018 with the Japan Patent Office. It is understood that the entire content thereof is incorporated herein by reference.

[Industrial Applicability]

The present disclosure is useful in providing a resistant starch that can be considered as "food product" under the "Specifications and Standards for Food, Food Additives, Etc." in Japan or equivalent standards in other countries and used in the manufacture of food products without deterioration in flavor.

**Claims**

1. A resistant starch, wherein supernatant obtained by suspending the resistant starch in ultrapure water at 33% and centrifuging for 10 minutes at 2000 × g exhibits an electrical conductivity of less than 350 µS/cm, and the resistant starch has retort resistance of 20% or greater.

2. The resistant starch of claim 1, wherein the retort resistance is 30% or greater.

3. The resistant starch of claim 1 or 2, wherein the retort resistance is 40% or greater.

4. The resistant starch of any one of claims 1 to 3, wherein thermal resistance is 90% or greater.

5. The resistant starch of any one of claims 1 to 4, wherein acid resistance is 58% or greater.

6. A composition comprising the resistant starch of any one of claims 1 to 5 for use as an alternative to a swelling suppressed modified starch.

7. A composition comprising the resistant starch of any one of claims 1 to 6 for the manufacture of a retort food product without compromising flavor.

8. A composition comprising the resistant starch of any one of claims 1 to 7 for use under a high temperature/high shearing condition.

9. A method of manufacturing a resistant starch, the method comprising the steps of:

mixing a raw material starch and an acid with at least one of a saccharide and an amino acid and subjecting the mixture to heating; and
washing a product with water after the step of subjecting the mixture to heating;
wherein the acid is a carboxylic acid.

10. The method of claim 9, wherein the raw material starch is mixed with the acid, the saccharide, and the amino acid.

11. The method of claim 9 or 10, wherein the acid, the saccharide, and/or the amino acid is provided as juice.

12. The method of any one of claims 9 to 11, wherein a pH of a mixture, when mixing the raw material starch, the acid, and the saccharide, is adjusted to a pH of 3.7 or greater before the step of subjecting the mixture to heating.

13. The method of any one of claims 9 to 11, wherein a pH of a mixture, when mixing the raw material starch, the acid, and the amino acid, is adjusted to a pH of 3 or greater before the step of subjecting the mixture to heating.

14. The method of any one of claims 9 to 11, wherein a pH of a mixture, when mixing the raw material starch, the acid, the saccharide, and the amino acid, is adjusted to a pH of 3 or greater before the step of subjecting the mixture to heating.

15. The method of any one of claims 9 to 14, further comprising the step of selecting a product, wherein supernatant obtained by suspending the product in ultrapure water at 33% and centrifuging the product for 10 minutes at 2000 $\times$ g exhibits an electrical conductivity of less than 350 $\mu$S/cm.

16. A resistant starch manufactured by the method of any one of claims 9 to 15.

17. A composition for manufacturing a resistant starch, comprising an acid.

18. The composition of claim 17, further comprising an amino acid, a saccharide, or a combination thereof.

19. The composition of claim 17 or 18, wherein the amino acid, the saccharide, and/or the acid is provided as juice.

20. A food product comprising the resistant starch of any one of claims 1 to 5 and 16.

21. The food product of claim 20, wherein the food product is yogurt, sauce, or a retort product.

22. A cosmetic product comprising the resistant starch of any one of claims 1 to 5 and 16.

23. An industrial product comprising the resistant starch of any one of claims 1 to 5 and 16.

# FIG.1

Juice type

```
  Acid                NaOH
   │                   │
   │         Adjusted pH (pH 5.9)
   │                   │
   ▼                   ▼
```

| Raw material starch | → | Mixture | → | Heating | → | Washing | → | Resistant starch |

Starch type       Amount of moisture    Temperature/time

**Figure 1  Flow chart of manufacturing method**

# FIG.2

Figure 2

# FIG.3

**Figure 3  State of starch particles after heating (microscope observation, scale 50 μm)**

## INTERNATIONAL SEARCH REPORT

| International application No. |
| --- |
| PCT/JP2019/042870 |

A. CLASSIFICATION OF SUBJECT MATTER
A23C 9/13(2006.01)i; A23L 3/00(2006.01)i; C08E 30/00(2006.01)i; A61Q
19/00(2006.01)i; A23L 5/00(2016.01)i; A23L 23/00(2016.01)i; A23L
29/00(2016.01)i; A23L 29/212(2016.01)i; A61K 8/73(2006.01)i
FI:      C08B30/00; A61K8/73; A61Q19/00; A23L5/00 N; A23L29/00;
         A23L29/212; A23C9/13; A23L23/00; A23L3/00 101C
According to International Patent Classification (IPC) or to both national classification and IPC

B. FIELDS SEARCHED

Minimum documentation searched (classification system followed by classification symbols)
A23C9/13; A23L3/00; C08B30/00; A61Q19/00; A23L5/00; A23L23/00; A23L29/00;
A23L29/212; A61K8/73

Documentation searched other than minimum documentation to the extent that such documents are included in the fields searched
Published examined utility model applications of Japan     1922-1996
Published unexamined utility model applications of Japan     1971-2020
Registered utility model specifications of Japan     1996-2020
Published registered utility model applications of Japan     1994-2020

Electronic data base consulted during the international search (name of data base and, where practicable, search terms used)
JSTPlus/ JMEDPlus/JST7580 (JDreamIII); CAplus (STN)

C. DOCUMENTS CONSIDERED TO BE RELEVANT

| Category* | Citation of document, with indication, where appropriate, of the relevant passages | Relevant to claim No. |
| --- | --- | --- |
| X | XIE, Xueju (Sherry), LIU, Qiang, "Development and physicochemical characterization of new resistant citrate starch from different corn starches.", Starch, 2004, 56, 364-370 pp.364-365 | 1-8 |
| Y | pp.364-365 | 12, 17, 20-23 |
| X | JP 9-278802 A (NAKANO VINEGAR CO., LTD.) 28.10.1997 (1997-10-28) claims, example 1 | 1-9, 16 |
| Y | claims, example 1 | 12, 17-18, 20-23 |
| X | WO 2009/110610 A1 (SANEI GEN FFI INC.) 11.09.2009 (2009-09-11) paragraphs [0145]-[0147] | 17-19 |
| A | JP 2005-171112 A (NIPPON STARCH CHEMICAL CO., LTD.) 30.06.2005 (2005-06-30) entire text | 1-23 |

☐ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 22 January 2020 (22.01.2020) | 04 February 2020 (04.02.2020) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**
Information on patent family members

International application No.

PCT/JP2019/042870

| Patent Documents referred in the Report | Publication Date | Patent Family | Publication Date |
|---|---|---|---|
| JP 9-278802 A | 28 Oct. 1997 | (Family: none) | |
| WO 2009/110610 A1 | 11 Sep. 2009 | US 2011/0020530 A1 paragraphs [0169]-[0170] EP 2251358 A1 | |
| JP 2005-171112 A | 30 Jun. 2005 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005171112 A
- JP 3513047 B
- JP 2016178892 A
- JP 2018206925 A

**Non-patent literature cited in the description**

- **KEIJI KAINUMA et al.** Studies on Structure and Physico-chemical Properties of Starch. *J. Jap. Soc. Starch Sci., Japanese Society of Starch Science,* 1975, vol. 22 (3), 66-71
- **YOSHIJIRO KIHARA et al.** Denpun ni Taisuru Arudehido no Sayo (I) [Action of aldehyde on starch (I). *Denpun Kogyo Gakkaishi, The Japanese Society of Applied Glycoscience,* 1962, vol. 10 (1), 1-6
- **KAZUHIRO OKUMA.** Denpun no Kako to Shokuhin Riyo [Starch modification and use in food products. *Journal of Applied Glycoscinece, The Japanese Society of Applied Glycoscience,* 2011, vol. 1 (1), 34-38
- **REIJI TAKAHASHI ; SAIWAI SHOBO.** *Denpun Sei-hin no Chishiki [Knowledge on starch products,* May 1996, 187-190
- **REIJI TAKAHASHI ; SAIWAI SHOBO.** *Denpun Sei-hin no Chishiki [Knowledge on starch products,* May 1996, 122-123
- **REIJI TAKAHASHI ; SAIWAI SHOBO.** *Denpun Sei-hin no Chishiki [Knowledge on starch products,* May 1996, 116-118
- Potatoes and starches/(starches)/Potato starch. Standard Tables of Food Composition. 2015
- Standard Tables of Food Composition. 2015
- **SHIGEO SUZUKI ; MICHITOKU NAKAMURA.** Den-pun Kagaku Jikkenho [Starch Science Experimental Method. Asakura Publishing, October 1979, 279-283